# EUROPÄISCHE PATENTSCHRIFT

(11) **EP 2 317 909 B1**
(45) Veröffentlichungstag und Bekanntmachung des Hinweises auf die Patenterteilung: **17.09.2014**
(21) Anmeldenummer: 09781580.7
(22) Anmeldetag: 06.08.2009
(51) Int. Cl.: A61B 5/00, A61B 19/00, G01N 33/48

(54) **MEDIZINISCHES SYSTEM MIT KOMPAKTEM BARCODELESER FÜR VERBRAUCHSARTIKEL**
MEDICAL SYSTEM COMPRISING COMPACT BARCODE READERS FOR CONSUMABLE ITEMS
SYSTÈME MÉDICAL AVEC LECTEUR DE CODES-BARRES COMPACT POUR CONSOMMABLES

(30) Priorität: 06.08.2008 EP 08161928
(43) Veröffentlichungstag der Anmeldung: 11.05.2011
(73) Patentinhaber: Roche Diagnostics GmbH, 68305 Mannheim (DE); F. Hoffmann-La Roche AG, 4070 Basel (CH)
(72) Erfinder: HAAR, Hans-Peter, 69168 Wiesloch (DE); KOCHERSCHEIDT, Gerrit, 68542 Heddesheim (DE)
(74) Vertreter: Stößel, Matthias
(86) Internationale Anmeldenummer: PCT/EP2009/060237
(87) Internationale Veröffentlichungsnummer: WO 2010/015689

(56) Entgegenhaltungen:
- WO-A-2006/099317
- WO-A-2008/010822
- JP-A- 2003 085 537
- KR-A- 20090 046 179
- US-A1- 2003 090 650
- US-A1- 2004 082 918
- US-A1- 2006 202 104
- US-A1- 2006 213 994
- LAN J ET AL: "Fingerprint imager based on a-si:h active-matrix photo-diode arrays" ELECTRON DEVICES MEETING, 2000. IEDM TECHNICAL DIGEST. INTERNATIONAL DECEMBER 10-13, 2000, PISCATAWAY, NJ, USA,IEEE, 10. Dezember 2000 (2000-12-10), Seiten 419-422, XP010531795 ISBN: 978-0-7803-6438-7

## Beschreibung

### Gebiet der Erfindung

Die Erfindung betrifft ein medizinisches System, welches mindestens ein medizinisches Gerät zur Durchführung einer medizinischen Funktion umfasst. Das medizinische Gerät ist eingerichtet, um mit mindestens einem medizinischen Verbrauchsartikel zusammenzuwirken. Derartige medizinische Systeme werden beispielsweise in der medizinischen Analytik, der medizinischen Diagnostik oder der medizinischen Therapeutik eingesetzt.

### Stand der Technik

Im Bereich der Medizin und Medizintechnik spielt die Verwendung von medizinischen Verbrauchsartikeln eine wesentliche Rolle. So werden beispielsweise in der medizinischen Diagnostik und Analytik und in der medizinischen Therapeutik in vielen Fällen medizinische Geräte eingesetzt, welche beispielsweise eine diagnostische, analytische oder therapeutische Funktion aufweisen und zur Durchführung dieser Funktion auf einen oder mehrere medizinische Verbrauchsartikel angewiesen sind.

Beispiele derartiger medizinischer Systeme sind medizinische Analysegeräte, welche zum quantitativen und/oder qualitativen Nachweis mindestens eines Analyten in einer Probe verwendet werden, beispielsweise zum Nachweis eines oder mehrerer Metaboliten in einer Körperflüssigkeit. Als Anwendungsbeispiel seien hier Glukosemessgeräte genannt, welche eingesetzt werden, um einen Glukosegehalt beispielsweise in Blut, interstitieller Flüssigkeit, Speichel oder Urin zu messen.

Derartige oder andere Analysegeräte verwenden in der Regel einen oder mehrere Testelemente, mittels derer der quantitative und/oder qualitative Nachweis des Analyten erfolgt. Beispielsweise können diese Testelemente ein oder mehrere Testfelder umfassen, welche bei Kontakt mit dem Analyten eine spezifische chemisch oder physikalisch nachweisbare Reaktion durchführen bzw. eine spezifische, messbare Veränderung erfahren. Dementsprechend können die Analysegeräte eingerichtet sein, um optisch, elektrochemisch oder auf anderem Wege mit den Testelementen die Analytkonzentration zu bestimmen. Die Testelemente können beispielsweise als Teströhrchen, Teststreifen, Testbänder, Testräder mit auf einer Oberseite und/oder einem Umfang angeordneten Testfeldern, faltbare Testpapiere mit mehreren Testfeldern oder in anderer Form vorliegen. Die Testelemente können dabei einzeln oder beispielsweise zu mehreren in Magazinform vorliegen, wobei im letzteren Fall auch ein Magazin mit den Testelementen als ein Verbrauchsartikel angesehen werden kann.

Ein anderes Beispiel derartiger medizinischer Systeme mit Verbrauchsartikeln sind Lanzettensysteme, bei welchen als medizinisches Gerät beispielsweise eine Stechhilfe fungiert. Diese Stechhilfe ist in der Regel eingerichtet, um mittels eines oder mehrerer Verbrauchsartikel in Form von Lanzetten eine Hautpartie eines Patienten zu perforieren, um beispielsweise eine Probe von Blut oder interstitieller Flüssigkeit zu generieren.

Weitere Beispiele derartiger medizinischer Systeme sind Medikationssysteme mit Dosiervorrichtungen. Derartige Dosiervorrichtungen arbeiten in der Regel sogar mit mehreren Arten medizinischer Verbrauchsartikel. So können zum einen beispielsweise Kartuschen oder andere Vorratsgefäße eines Medikaments verwendet werden, welches mittels der Dosiervorrichtung dosiert wird. In diesem Fall kann das Medikament selbst und/oder das Medikament mit dem entsprechenden Gefäß (beispielsweise der Kartusche) als Verbrauchsartikel angesehen werden. Ein Beispiel derartiger Dosiervorrichtungen sind Medikationspumpen, wie beispielsweise Insulinpumpen. Diese Dosiervorrichtungen erfordern jedoch in der Regel weiterhin weitere Arten von medizinischen Verbrauchsartikeln, insbesondere beispielsweise Katheter.

Aus US 2004/0082918 A1 ist ein Dosiersystem für Medikamente bekannt. Dieses umfasst eine Station mit einem Barcode. Eine Spritze mit einer Nadel kann aufgenommen werden, wobei die Spritze ebenfalls einen Barcode aufweist. Ein gleitbar gelagertes Scannermodul ist vorgesehen, um die Barcodes auszulesen. Unter anderem wird die Verwendung eines optischen Faserbündels zum Auslesen der Barcodes beschrieben.

Zahlreiche weitere Arten medizinischer Systeme dieser Art, mit einem medizinischen Gerät und mindestens einem Verbrauchsartikel, sind bekannt. Eine Herausforderung dieser medizinischen Systeme besteht in der Praxis darin, dass das medizinische Gerät, um korrekt zur Durchführung seiner medizinischen Funktion mit dem Verbrauchsartikel zusammenwirken zu können, Informationen benötigt, welche wechseln können. So können sich beispielsweise Testelemente von Charge zu Charge unterscheiden, so dass zur korrekten Auswertung des quantitativen und/oder qualitativen Nachweises des mindestens einen Analyten in der Probe eine chargenspezifische Information benötigt werden kann. Dies kann beispielsweise eine Information darüber sein, wie sich die optischen Lumineszenzoder Absorptionseigenschaften, d.h. z.B. die Lumineszenz oder Farbe eines Testfeldes eines Testelements, mit der Analytkonzentration ändert. Auch elektrochemische Auswertungsinformationen können umfasst sein. Dabei werden beispielsweise Stromverläufe und/oder elektrische Potentiale gemessen.

Bei Lanzettensystemen, beispielsweise Lanzettensystemen mit Verbrauchsartikeln in Form eines Lanzettenmagazins mit mehreren Lanzetten oder in Form einer einzelnen Lanzette, kann beispielsweise eine Stechhilfe eine Information darüber benötigen, ob eine korrekte Art von Verbrauchsartikel in die Stechhilfe eingesetzt wurde, beispielsweise eine Lanzette eines korrekten Herstellers oder Typs. Allgemein können derartige Informationen bei dieser Art von Verbrauchsartikeln oder anderen Arten von Verbrauchsartikeln beispielsweise auch zu einer Fälschungssicherung verwendet werden, um Artikel eines korrekten oder autorisierten Herstellers von "gefälschten" Verbrauchsartikeln zu unterscheiden. Letzteres kann, neben der Vermeidung wirtschaftlicher Schäden, die Gefahr gesundheitlicher Schäden durch gefälschte Medizinprodukte stark verringern.

Bei medizinischen Systemen mit Dosiervorrichtungen, beispielsweise Insulinpumpen, kann beispielsweise eine Information über die Art und/oder den Inhalt einer Kartusche eines Medikaments erforderlich sein. Werden Katheter bzw. Kanülen verwendet, um das Medikament zu dosieren, so kann beispielsweise ein Füllvolumen des Katheters erforderlich sein, um ein korrektes anfängliches Befüllen oder Fluten ("Priming") des Katheters zu gewährleisten.

Dies sind nur einige Beispiele von Informationen, die bei derartigen medizinischen Systemen ausgetauscht werden können oder müssen. Zur Lösung dieser Problematik bestehen im Stand der Technik verschiedene Möglichkeiten. So wird bei handelsüblichen Glukosemessgeräten beispielsweise jeder Charge neuer Testelemente ein Informationsträger beigefügt, beispielsweise ein so genannter ROM-key. Vom Patienten wird verlangt, dass dieser, vor Benutzung der neuen Charge, diesen ROM-key in das Analysegerät eingibt, so dass korrekte Informationen für die Auswertung der Messung verwendet werden können. Diese Technik ist jedoch grundsätzlich mit dem Risiko verbunden, dass, gerade bei älteren Patienten oder Kindern, bei Verwendung einer neuen Charge am Teststreifen der Austausch des ROM-keys unterbleibt. Dies kann, da in diesem Fall möglicherweise inkorrekte Messergebnisse ausgegeben werden, Folgen hinsichtlich einer auf den fehlerhaften Messergebnissen basierenden fehlerhaften Medikation haben.

Aus dem Stand der Technik sind daher verschiedene medizinische Systeme bekannt, bei welchen unmittelbar auf dem Verbrauchsmaterial, also nicht als separater Informationsträger, sondern fest mit dem Verbrauchsmaterial verbunden, ein derartiger Informationsträger vorgesehen ist. Da diese Informationsträger aufgrund eines im medizinischen Bereich stetig wachsenden Kostendrucks kostengünstig und darüber hinaus sehr klein ausgestaltet werden müssen, scheiden bekannte elektronische Informationsträger (wie beispielsweise Hochfrequenzetiketten) in vielen Fällen aus.

Es sind daher medizinische Systeme bekannt, bei welchen zwei- oder dreidimensionale optische Codes auf medizinische Verbrauchsartikel aufgebracht werden, welche mit einem entsprechenden optischen Codeleser des medizinischen Geräts eingelesen werden können. Derartige Systeme werden beispielsweise in US 6,588,670 B2 beschrieben. Auch in US 4,476,149 oder in US 6,168,957 werden Teststreifen beschrieben, welche mit entsprechenden Barcodes als optische Codes ausgestattet sind. Auf derartige optische Codes kann im Folgenden beispielhaft Bezug genommen werden.

Eine Schwierigkeit bei derartigen optischen Codes besteht jedoch darin, dass, insbesondere in medizinischen Handgeräten, der für den Codeleser zur Verfügung stehende Bauraum äußerst knapp bemessen ist. Zudem müssen die Codeleser sehr leicht aufgebaut sein und müssen in Massenproduktion kostengünstig fertigbar sein. Aus dem Stand der Technik sind zahlreiche Codeleser zum Auslesen optischer Codes bekannt. Beispiele derartiger Codeleser sind in US 2006/0213994 A1 für DNA-Microarray-Scanner oder aus US 7,175,091 B2 für Scheckkartenleser bekannt.

Aus anderen Bereichen der Technik sind optisch auflösende Nahsensoren, insbesondere in Form so genannter Contact-Imaging-Sensors (CIS), bekannt. So beschreibt beispielsweise US 2008/0088731 A1 einen dünnen Bildsensor, bei welchem mittels eines Mikrolinsen-Arrays ein Bild des Objekts auf einem Bildsensor erzeugt wird. US 2006/0202104 A1 beschreibt einen Fingerabdruck-Sensor, bei welchem ebenfalls mit einem Mikrolinsen-Array eine Projektion auf eine CCD/CMOS-Struktur erfolgt.

Derartige Codeleser oder Bildsensoren weisen jedoch für den Einsatz in medizinischen Systemen der oben beschriebenen Art eine Mehrzahl von Nachteilen auf. So erfordern viele dieser Sensoren, insbesondere aufgrund der Verwendung von Mikrolinsen-Arrays, einen vergleichsweise großen Bauraum. Zudem ist der Herstellaufwand für Mikrolinsensysteme beträchtlich, und die Auflösung derartiger Mikrolinsensysteme ist in der Regel für sehr kleine optische Codes, wie sie beispielsweise auf Teststreifen erforderlich sind, ungenügend.

Eine besondere Problematik stellt hierbei insbesondere die Beleuchtung dar, welche für viele Codeleser erforderlich ist. Eine Beleuchtung durch den medizinischen Verbrauchsartikel hindurch, wie sie beispielsweise in US 2006/0213994 A1 beschrieben wird, ist aufgrund der Beschaffenheit vieler Verbrauchsartikel, wie beispielsweise nicht-transparenter Teststreifen, in zahlreichen Fällen nicht realisierbar. Auch eine refle-ktive Beleuchtung der optischen Codes scheidet für viele medizinische Systeme aus, da der zur Verfügung stehende Bauraum es bei den bekannten Systemen und Beleuchtungstechniken nicht erlaubt, die Fläche des optischen Codes ausreichend zu beleuchten.

### Aufgabe der Erfindung

Es ist daher eine Aufgabe der vorliegenden Erfindung, ein medizinisches System bereitzustellen, welches die Nachteile der oben beschriebenen medizinischen Systeme zumindest weitgehend vermeidet. Das medizinische System soll einen zuverlässigen, Bauraum sparenden und kostengünstigen Austausch von Informationen zwischen mindestens einem medizinischen Verbrauchsartikel und einem medizinischen Gerät der oben beschriebenen Art ermöglichen.

### Offenbarung der Erfindung

Diese Aufgabe wird durch ein medizinisches System mit den Merkmalen des unabhängigen Anspruchs gelöst. Vorteilhafte Weiterbildungen der Erfindung, welche einzeln oder in Kombination realisierbar sind, sind in den abhängigen Ansprüchen dargestellt.

Das medizinische System umfasst mindestens ein medizinisches Gerät zur Durchführung mindestens einer medizinischen Funktion. Wie oben dargelegt, kann es sich bei dieser medizinischen Funktion um eine beliebige, üblicherweise im Bereich der Medizin oder Medizintechnik benötigte Funktion handeln, insbesondere eine diagnostische und/oder analytische Funktion und/oder eine therapeutische Funktion. Unter einer diagnostischen Funktion kann dabei eine Funktion verstanden werden, welche auf die Ermittlungen mindestens eines medizinischen Zustandes eines Patienten abzielt. Unter einer analytischen Funktion kann eine nahezu beliebige Messfunktion verstanden werden, welche auf die Messung einer oder mehrerer Parameter, beispielsweise einer Probe, abzielt. Als Beispiel kann hier ein qualitativer und/oder quantitativer Nachweis mindestens eines Analyten in einer flüssigen, festen oder gasförmigen Probe genannt werden. Unter einer therapeutischen Funktion kann eine Funktion verstanden werden, welche auf eine gezielte Beeinflussung eines Körperzustandes eines Patienten gerichtet ist. Diese Beeinflussung wird in der Regel auf eine Verbesserung des Körperzustandes, also beispielsweise eine Heilung, abzielen. Auch andere Arten der Beeinflussung sind jedoch möglich, beispielsweise Beeinflussungen aus kosmetischen Gründen. Als Beispiele therapeutischer Funktionen können Medikationen genannt werden, beispielsweise Verabreichungen von Medikamenten durch Injektion. Auch andere Arten therapeutischer Funktionen sind jedoch grundsätzlich möglich, beispielsweise Akupressuren und/oder Akupunkturen. Für weitere Beispiele medizinischer Geräte, auf welche auch im Rahmen der vorliegenden Erfindung Bezug genommen werden kann, kann auf die obige Beschreibung des Standes der Technik verwiesen werden. Das medizinische Gerät kann auch mehrere derartiger medizinischer Funktionen durchführen, beispielsweise Kombinationen von diagnostischen und therapeutischen Funktionen. Das medizinische Gerät kann zur Durchführung dieser mindestens einen medizinischen Funktion entsprechend durch eine oder mehrere mechanische Vorrichtungen und/oder eine oder mehrere elektronische Vorrichtungen und/oder eine oder mehrere, gegebenenfalls programmtechnisch eingerichtete, Datenverarbeitungsvorrichtungen ausgestaltet sein.

Das medizinische Gerät ist eingerichtet, um zur Durchführung der mindestens einen medizinischen Funktion mit mindestens einem Verbrauchsartikel zusammenzuwirken. Unter einem Zusammenwirken ist dabei ein funktionelles Zusammenwirken zu verstehen, welches nicht notwendigerweise eine physikalische Verbindung zwischen medizinischem Gerät und Verbrauchsartikel erfordert. Eine derartige physikalische Verbindung kann jedoch gleichwohl gegeben sein, beispielsweise in Form einer mechanischen und/oder elektrischen Verbindung. So kann beispielsweise das medizinische Gerät eine Haltevorrichtung und/oder eine Aufnahme und/oder eine Positionierungsvorrichtung umfassen, welche den mindestens einen medizinischen Verbrauchsartikel aufnehmen bzw. haltern bzw. positionieren.

Der medizinische Verbrauchsartikel soll dabei eingerichtet sein, um die medizinische Funktion des medizinischen Geräts zu ermöglichen oder zumindest zu unterstützen. In anderen Worten kann der Verbrauchsartikel eingerichtet sein, um komplementär mit dem medizinischen Gerät zusammenzuwirken, um beispielsweise die analytische und/oder diagnostische und/oder therapeutische Funktion zu gewährleisten. Unter dem Begriff "Verbrauchsartikel" ist dabei ein Artikel zu verstehen, welcher vorzugsweise großtechnisch hergestellt werden kann. Dieser Verbrauchsartikel soll nach Belieben austauschbar sein, wohingegen das medizinische Gerät in der Regel für eine Mehrfachverwendung geeignet ist. So kann der Verbrauchsartikel beispielsweise für einen einmaligen Gebrauch vorgesehen sein oder lediglich für eine begrenzte Anzahl von Nutzungen.

Das medizinische Gerät umfasst weiterhin mindestens einen Codeleser zum Auslesen mindestens einer Information eines optischen Codes auf dem medizinischen Verbrauchsartikel. Unter einem optischen Code ist dabei ein mittels Licht im sichtbaren und/oder infraroten und/oder ultravioletten Spektralbereich des elektromagnetischen Spektrums auslesbarer Informationsträger zu verstehen, insbesondere ein zwei- und/oder dreidimensionaler Barcode. Alternativ oder zusätzlich sind auch zahlreiche andere Arten optischer Codes realisierbar, beispielsweise Barcodes, Graustufencodes oder ähnliche Arten von optischen Codes oder Kombinationen der genannten und/oder anderer Arten von Codes. Der optische Code kann beispielsweise auf einer Oberfläche des medizinischen Verbrauchsartikels aufgebracht sein, wobei unter einer Oberfläche sinngemäß auch zu verstehen ist, dass der optische Code von einer zumindest teilweise optisch transparenten Beschichtung bedeckt ist und somit noch ganz oder teilweise optisch auslesbar ist. In jedem Fall soll der optische Code von außen mittels elektromagnetischer Strahlung einer geeigneten Wellenlänge auslesbar sein.

Der Codeleser des medizinischen Geräts umfasst mindestens eine optische mehrkanalige Auswerteeinheit, welche im Folgenden kurz als Bildsensor bezeichnet wird. Der Bildsensor umfasst eine Mehrzahl von Sensoren. Diese Sensoren, welche beispielsweise ein- oder zweidimensional angeordnet sein können, beispielsweise in einem ein- oder zweidimensionalen Sensor-Array, sollen zur Aufnahme optischer Signale geeignet sein.

Neben dem Bildsensor umfasst der Codeleser weiterhin mindestens eine lichtoptische Faserplatte. Unter einer lichtoptischen Faserplatte ist dabei ein Element zu verstehen, welches eine Mehrzahl optischer Fasern umfasst, welche vorzugsweise alle parallel oder im Wesentlichen parallel ausgerichtet sind. Unter "im Wesentlichen parallel" können dabei jedoch auch Ausrichtungen verstanden werden, welche von einer Parallelität abweichen, beispielsweise um nicht mehr als 20°. Unter einer optischen Faser ist dabei im Rahmen der vorliegenden Erfindung ein für Licht im sichtbaren und/oder infraroten und/oder ultravioletten Spektralbereich transparentes Element zu verstehen, welches als Lichtleiter wirkt, insbesondere aufgrund von innerer Totalreflexion. Die lichtoptische Faserplatte kann derartige optische Fasern beispielsweise als Bündel umfassen, wobei die optischen Fasern beispielsweise miteinander verschmolzen, vergossen oder verklebt sind.

Vorzugsweise sind diese optischen Fasern in der lichtoptischen Faserplatte derart angeordnet, dass diese zumindest in einer Dimension als dichteste Packung arrangiert sind, so dass der Bauraum durch die lichtoptischen Fasern zu einem höchstmöglichen Grad ausgenutzt wird. So können beispielsweise Fasern mit rundem Querschnitt in einer hexagonalen Anordnung angeordnet sein. Auch andere Anordnungen sind jedoch möglich. Die lichtoptische Faserplatte ist als ebene Platte ausgestaltet, also als scheibenförmiges Element einer vorgegebenen Dicke, welches eine laterale Erstreckung aufweist, die vorzugsweise größer ist als die Dicke dieser Faserplatte. Beispielsweise kann es sich um eine Platte mit polygonalem, rundem oder andersartigem Querschnitt handeln.

Die lichtoptische Faserplatte ist eingerichtet, um Licht von einer ersten Ebene der lichtoptischen Faserplatte zu einer zweiten Ebene der lichtoptischen Faserplatte zu transportieren, ohne hierbei das Bild nennenswert zu verändern. Jedes Ende einer lichtoptischen Faser wirkt zumindest näherungsweise als Punktlichtquelle, welche die Lichtverhältnisse an ihrem gegenüberliegenden Ende im Wesentlichen reproduziert.

Der Codeleser ist dabei derart aufgebaut, dass die lichtoptische Faserplatte derart angeordnet ist, dass ein Bild des optischen Codes zu dem Bildsensor geleitet wird. Die lichtoptische Faserplatte ist also zwischen dem Bildsensor und dem optischen Code angeordnet, so dass der optische Code durch die lichtoptische Faserplatte gleichsam von der dem optischen Code benachbarten Ebene der lichtoptischen Faserplatte auf die gegenüberliegende Ebene dieser lichtoptischen Faserplatte versetzt wird, um dort von dem Bildsensor aufgenommen zu werden.

Der Bildsensor und die lichtoptische Faserplatte können dabei eine Einheit bilden, welche auch sehr kompakt ausgestaltet sein kann. So können der Bildsensor und die lichtoptische Faserplatte beispielsweise durch eine mechanische und/oder formschlüssige und/oder kraftschlüssige Verbindung miteinander zu der Einheit verbunden sein. Bildsensor und lichtoptische Faserplatte, insbesondere die aus diesen Elementen gebildete Einheit, können gemeinsam eine Dicke von weniger als 5 mm aufweisen.

Die Sensoren des Bildsensors können eine lineare Anordnung, also eine eindimensionale Anordnung, oder auch eine zweidimensionale Anordnung bilden, beispielsweise eine Matrixanordnung. Insbesondere kann der Bildsensor einen oder mehrere der folgenden Sensoren umfassen: einen CCD-Sensor, insbesondere ein CCD-Array; einen CMOS-Sensor, insbesondere ein CMOS-Array, einen Photodiodensensor, insbesondere ein Photodioden-Array; einen organischen Photodetektor, insbesondere eine organische Photodiode, insbesondere ein Array derartiger organischer Photodetektoren. Grundsätzlich sind jedoch auch andere Arten bildgebender Sensoren einsetzbar, die auf anderen physikalischen Prinzipien basieren, oder Kombinationen der genannten und/oder weiterer bildgebender Sensoren.

Besonders bevorzugt ist es, wenn die lichtoptische Faserplatte unmittelbar auf den Bildsensor aufgebracht wird. Allgemein ist es besonders bevorzugt, wenn der Abstand zwischen dem Bildsensor und der lichtoptischen Faserplatte kleiner ist als ein Abstand zwischen benachbarten Sensoren des Bildsensors. Da die lichtoptische Faserplatte auf ihrer dem Bildsensor zugewandten Seite die Lichtverhältnisse auf ihrer dem Bildsensor abgewandten Seite im Wesentlichen identisch wiedergibt, ist diese Anordnung gleichbedeutend zu einer Anordnung, bei welcher der Bildsensor im Wesentlichen unmittelbar auf dem Code aufliegt. Im Gegensatz zu letzterer Anordnung bietet die lichtoptische Faserplatte jedoch, wie unten näher erläutert wird, die Möglichkeit, eine Lichteinkopplung durch die lichtoptische Faserplatte zu bewirken, so dass der Code effizient beleuchtet werden kann.

Die lichtoptische Faserplatte kann grundsätzlich aus einem beliebigen transparenten Material hergestellt sein. Insbesondere lassen sich Kunststoffmaterialien und/oder Gläser verwenden. Diese können auch in einer Kern-Mantel-Struktur (Core-Cladding-Struktur) ausgestaltet sein, wobei beispielsweise die Mäntel der einzelnen optischen Fasern miteinander verschmolzen oder verklebt sind und eine gemeinsame Matrix der lichtoptischen Faserplatte bilden, in welche die eigentlichen Faserkerne eingebettet sind. Besonders bevorzugt ist es, wenn die optischen Fasern und/oder deren Kerne, welche in der optischen Faserplatte eingebettet sind, einen Durchmesser von weniger als 100 µm aufweisen, vorzugsweise 80 µm oder weniger.

Der Codeleser weist weiterhin mindestens eine Beleuchtungseinrichtung auf, also eine Einrichtung, welche ausgestaltet ist, um den optischen Code des Verbrauchsartikels zu beleuchten. Diese Beleuchtung erfolgt einseitig, von derselben Seite, auf welcher auch der Bildsensor vorgesehen ist. In diesem Fall kann, im Gegensatz zu Durchleuchtungsvorrichtungen, der gesamte Codeleser sehr kompakt ausgestaltet sein, wobei der medizinische Verbrauchsartikel lediglich einseitig an den Codeleser herangeführt werden muss. Diese Beleuchtung von der Detektorseite her, also von der Seite des Bildsensors her, bietet somit Vorteile hinsichtlich des Bauraums. Diese Vorteile werden, wie oben beschrieben, durch die Verwendung der lichtoptischen Faserplatte unterstützt, da eine Beleuchtung durch die lichtoptische Faserplatte hindurch erfolgen kann, ohne die Qualität der Bildaufnahme des Codes oder den Bauraumbedarf negativ zu beeinträchtigen. Zusätzlich sind jedoch auch andere Arten der Beleuchtung möglich.

Die Beleuchtungseinrichtung umfasst dementsprechend mindestens eine Lichtquelle, welche auf verschiedene Arten angeordnet sein kann. Auch mehrere Lichtquellen unterschiedlicher Anordnung und/oder unterschiedlicher spektraler Eigenschaften können vorgesehen sein. So kann zum einen die Lichtquelle, wie aus dem Stand der Technik bekannt, als Durchlicht-Lichtquelle verwendet werden und kann eingerichtet sein, um den medizinischen Verbrauchsartikel im Bereich des optischen Codes zu durchleuchten. In diesem Falle sollte der Verbrauchsartikel in diesem Bereich zumindest teilweise für die verwendete Wellenlänge transparent sein oder zumindest das Licht der Lichtquelle zumindest teilweise zum optischen Code transportieren können. Verschiedene Ausführungsbeispiele derartiger medizinischer Verbrauchsartikel bzw. von Trägermaterialien, welche diesen Anforderungen genügen, werden unten beschrieben. Alternativ oder zusätzlich ist jedoch, wie unten näher ausgeführt wird, auch eine Lichtquelle für eine einseitige Beleuchtung möglich und im Rahmen der vorliegenden Erfindung bevorzugt.

Wie oben dargelegt, kann zur Beleuchtung Licht einer Wellenlänge im sichtbaren und/oder infraroten und/oder ultravioletten Spektralbereich eingesetzt werden. Insbesondere bietet sich der Spektralbereich im Bereich zwischen 300 nm und 3000 nm an. Hierfür können geeignete Lichtquellen eingesetzt werden, insbesondere ein oder mehrere Leuchtdioden auf anorganischer und/oder organischer Halbleiterbasis, insbesondere auch als Leuchtdioden-Arrays. Auch andere Arten von Lichtquellen können eingesetzt werden, beispielsweise Laser, insbesondere Halbleiterlaser.

Die Beleuchtungseinrichtung kann als monochromatische Beleuchtungseinrichtung ausgestaltet sein, kann jedoch auch eingerichtet sein, um den medizinischen Verbrauchsartikel mit Licht verschiedener Wellenlängen zu beleuchten. Unter "verschiedenen Wellenlängen" sind dabei spektrale Eigenschaften zu verstehen, bei welchen das spektrale Profil des Lichts zumindest nicht vollständig übereinstimmt, beispielsweise indem die Peakwellenlängen voneinander verschieden sind. Insbesondere kann die Beleuchtungseinrichtung eingerichtet sein, um den medizinischen Verbrauchsartikel, dort insbesondere den optischen Code, gleichzeitig oder auch sequentiell, d.h. zu unterschiedlichen Zeitpunkten, mit Licht verschiedener Wellenlängen zu beleuchten. Auch eine gleichzeitige Beleuchtung mit Licht verschiedener Wellenlängen ist jedoch grundsätzlich möglich, beispielsweise einhergehend mit einer entsprechenden spektralen Trennung durch den Bildsensor und/oder ein anderes wellenlängenselektives Element, beispielsweise einen oder mehrere Filter und/oder einen oder mehrere dichroitische Spiegel. Verschiedene Ausgestaltungen sind möglich. Weiterhin kann, alternativ oder zusätzlich, der Codeleser auch eingerichtet sein, um ein Abfrage-Antwort-Schema durchzuführen. So kann der Codeleser beispielsweise eingerichtet sein, um das Bild des optischen Codes zeitverzögert zu einer Beleuchtung durch die Beleuchtungseinrichtung aufzunehmen. So kann beispielsweise eine Schaltung vorgesehen sein, bei welcher eine Signalaufnahme durch den Bildsensor erst beginnt, wenn die Beleuchtung durch die Lichtquelle ausgeschaltet ist. Beispielsweise kann ein gepulstes Schema verwendet werden, mit einem Anregungspuls von beispielsweise weniger als 100 µsec. Ein Messbeginn kann dann um einen Zeitversatz, beispielsweise von 200 µsec, nach Ende des Anregungspulses versetzt erfolgen. Ein derartiges Abfrage-Antwort-Pulsschema kann insbesondere bei lumineszierenden Codes verwendet werden, beispielsweise bei Codes, welche ein oder mehrere Module mit fluoreszierenden und/oder phosphoreszierenden Medien umfassen z.B. aus der Gruppe der Komplexe seltener Erden wie z.B. Europium.

Wie oben dargestellt, erfolgt die Beleuchtung durch die Beleuchtungseinrichtung einseitig, d.h. dass die Beleuchtung von derselben Seite des medizinischen Verbrauchsartikels her erfolgt wie die Beobachtung durch die lichtoptische Faserplatte und den Bildsensor. Dies kann auf verschiedene Weisen realisiert werden. Insbesondere kann die Beleuchtung durch die lichtoptische Faserplatte hindurch erfolgen. Beispielsweise kann dies realisiert werden, indem die lichtoptische Faserplatte von der Seite her, also beispielsweise unter 90° zur Orientierung der optischen Fasern in der lichtoptischen Faserplatte oder unter einem Winkel, welcher vorzugsweise um nicht mehr als 20-30° von einem rechten Winkel abweicht, von mindestens einer Lichtquelle der Beleuchtungseinrichtung beleuchtet wird.

Alternativ oder zusätzlich kann die Beleuchtung jedoch auch im Wesentlichen parallel zu den optischen Fasern der lichtoptischen Faserplatte erfolgen. Dies kann beispielsweise dadurch geschehen, dass seitlich an dem Bildsensor vorbei durch die lichtoptische Faserplatte hindurch beleuchtet wird. Alternativ oder zusätzlich kann jedoch auch durch den Bildsensor hindurch beleuchtet werden, so dass das Anregungslicht zunächst den Bildsensor und dann die lichtoptische Faserplatte durchdringt. Dies kann beispielsweise dann geschehen, wenn der Bildsensor zumindest teilweise transparent ist für das verwendete Anregungslicht der Lichtquelle der Beleuchtungseinrichtung. Zu diesem Zweck können beispielsweise in dem Bildsensor entsprechende Öffnungen vorgesehen sein, durch welche das Anregungslicht treten kann, um dann durch die lichtoptische Faserplatte zu dem medizinischen Verbrauchsartikel und dem Code zu gelangen. Alternativ oder zusätzlich kann jedoch auch das Material des Bildsensors selbst zumindest teilweise transparent für das Anregungslicht sein. Beispielsweise kann der Bildsensor ein Halbleitermaterial (z.B. Silizium) mit einer Bandlücke aufweisen, wobei die Lichtquelle eingerichtet ist, um Licht mit einer geringeren Energie als die Bandlücke zu emittieren. In anderen Worten ist der Bildsensor derart eingerichtet, dass dieser das Anregungslicht der Lichtquelle im Wesentlichen nicht oder nur unwesentlich absorbiert, beispielsweise zu nicht mehr als 20%. Bei Silizium kann dies beispielsweise dadurch geschehen, dass Licht mit einer Wellenlänge von mehr als 1000 nm verwendet wird. Sollte die Energie dieses Anregungslichts nicht ausreichen, um den optischen Code auszulesen oder genügend zu beleuchten, so kann der optische Code entsprechende Lichtkonverter umfassen, welche beispielsweise aus den längerwelligen Photonen des Anregungslichts der Lichtquelle kürzerwellige Photonen erzeugen, beispielsweise im Rahmen von Multiphotonenprozessen.

Alternativ oder zusätzlich zu einer Beleuchtung durch die lichtoptische Faserplatte hindurch kann die Beleuchtungseinrichtung jedoch auch eingerichtet sein, um den optischen Code von der Seite her, also vorzugsweise im Wesentlichen senkrecht zu einer Blickrichtung der lichtoptischen Faserplatte, zu beleuchten. Dies kann beispielsweise durch ein Trägermaterial des medizinischen Verbrauchsartikels im Bereich des optischen Codes hindurch erfolgen, beispielsweise wiederum durch ein das Anregungslicht der Lichtquelle der Beleuchtungseinrichtung leitendes oder für dieses Anregungslicht transparentes Trägermaterial.

Zusätzlich besteht weiterhin, wie oben dargestellt, natürlich auch die Möglichkeit, den medizinischen Verbrauchsartikel zumindest im Bereich des optischen Codes von der Rückseite her zu beleuchten, also von der der lichtoptischen Faserplatte und dem Bildsensor abgewandten Seite. In diesem Fall ist ein zumindest teilweise transparentes oder lichtleitendes Trägermaterial in diesem Bereich des medizinischen Verbrauchsartikels bevorzugt. Diese Art der Beleuchtung, welche zusätzlich zu den anderen Beleuchtungsarten eingesetzt werden kann, kann überwiegend für eine absorptive Detektion des optischen Codes genutzt werden, jedoch auch beispielsweise für eine Lumineszenzdetektion.

Der Bildsensor kann weiterhin mindestens zwei Bereiche mit unterschiedlicher spektraler Empfindlichkeit aufweisen. Beispielsweise kann der Bildsensor unterschiedliche Sensoren umfassen, welche unterschiedliche spektrale Empfindlichkeit aufweisen. Auf diese Weise können beispielsweise mehrere Lichtwellenlängen, welche von dem optischen Code reflektiert oder emittiert oder in sonstiger Weise ausgehen, zur Detektion genutzt werden. Beispielsweise können diese unterschiedlichen Empfindlichkeiten durch intrinsisch unterschiedliche Empfindlichkeiten der Sensoren erreicht werden, oder es können ein oder mehrere Filter zur Erzeugung dieser unterschiedlichen spektralen Empfindlichkeiten verwendet werden. Allgemein kann der Codeleser mindestens einen optischen Filter, insbesondere einen Kantenfilter und/oder einen Interferenzfilter, aufweisen.

Der optische Code des medizinischen Verbrauchsartikels kann insbesondere aus einer Mehrzahl von optisch lesbaren Modulen als kleinster Informationseinheit zusammengesetzt sein. Diese kleinste Informationseinheit kann beispielsweise einem "bit" entsprechen. Wie oben beschrieben, ist der Code zweidimensional aufgebaut, so dass ein Modul in zwei Dimensionen die kleinste, durch den Codeleser aufzulösende Einheit darstellt.

Es ist besonders bevorzugt, wenn pro Modul in jeder Dimension, die zur Darstellung von Informationen genutzt wird, mindestens drei optische Fasern vorgesehen sind. Bei einem zweidimensionalen Code sollten daher pro Modul vorzugsweise mindestens neun Fasern vorgesehen sein. Bei einem zweidimensionalen Code können die Module beispielsweise rund oder rechteckig, insbesondere quadratisch, ausgestaltet sein, wobei die kleinste Kantenlänge eines Quadrats der Dimension eines Moduls entspricht. Beispielsweise können Module mit einer Breite von 300 µm verwendet werden.

Die lichtoptische Faserplatte ist dabei derart ausgestaltet und derart relativ zu dem optischen Code positioniert, dass diese eine hinreichende Auflösung zur Auslesung des optischen Codes bereitstellt. Dies wird dadurch gewährleistet, dass das medizinische Gerät und/oder der Codeleser und der medizinische Verbrauchsartikel derart zueinander positioniert sind, dass ein Abstand zwischen der lichtoptischen Faserplatte und dem optischen Code kleiner ist als ein Abstand zwischen den Mittelpunkten benachbarter Module des Codes. In anderen Worten soll die mittlere freie Weglänge, welche Photonen von den Modulen des Codes hin zur lichtoptischen Faserplatte zurückzulegen haben, kleiner sein als der Abstand zwischen den Mittelpunkten benachbarter Module, also der aufzulösenden Dimensionen des optischen Codes.

Allgemein kann das medizinische System eine Positionierungsvorrichtung umfassen. Diese Positionierungsvorrichtung kann eingerichtet sein, um den Verbrauchsartikel und das medizinische Gerät relativ zueinander zu positionieren, beispielsweise um ein optimales Lesen des optischen Codes zu ermöglichen. Beispielsweise kann die Positionierungsvorrichtung eingerichtet sein, um das medizinische Gerät und den Verbrauchsartikel relativ zueinander derart zu positionieren, dass in einer Lesestellung die oben beschriebene Bedingung bezüglich des Abstandes zwischen der lichtoptischen Faserplatte und dem optischen Code und des Abstandes zwischen den Mittelpunkten benachbarter Module des Codes gegeben ist. Alternativ oder zusätzlich kann die Positionierungsvorrichtung beispielsweise auch eingerichtet sein, um stets einen gleichbleibenden Abstand zwischen dem Verbrauchsartikel und der lichtoptischen Faserplatte, einschließlich beispielsweise eines optionalen Toleranzbereiches für Fehlpositionierungen, zu gewährleisten.

Beispielsweise kann die Positionierungsvorrichtung eingerichtet sein, um stets einen gewissen Mindestabstand zwischen der lichtoptischen Faserplatte und dem Verbrauchsartikel zu gewährleisten. Dies kann insbesondere bei Testelementen, wie beispielsweise Teststreifen, von Vorteil sein, welche insbesondere in das medizinische Gerät eingeschoben werden können. Erfolgt das Einschieben mit einem zu geringen Abstand zwischen dem Testelement und der lichtoptischen Faserplatte, so kann dies beispielsweise zu einer Beschädigung (beispielsweise einer Abnutzung und/oder einem Verkratzen) der lichtoptischen Faserplatte und/oder zu einer Verschmutzung derselben, beispielsweise durch an dem Testelement anhaftendes Blut, führen. Durch eine geeignete Ausgestaltung der Positionierungsvorrichtung kann dies verhindert werden.

So kann die Positionierungsvorrichtung beispielsweise mindestens einen Abstandshalter umfassen, welcher stets einen Mindestabstand zwischen dem medizinischen Verbrauchsartikel, beispielsweise dem Testelement, und dem Codeleser, beispielsweise der lichtoptischen Faserplatte des Codelesers, sicherstellt. Dieser Abstandshalter kann beispielsweise mindestens eine Schiene (insbesondere eine Abstandsschiene), mindestens einen Aufsatz, mindestens eine Führung, mindestens ein Distanzplättchen und/oder mindestens einen Distanzring oder auch Kombinationen der genannten Elemente und/oder weiterer Elemente umfassen.

Um andererseits zu gewährleisten, dass der Abstand zwischen dem medizinischen Verbrauchsartikel und dem Codeleser, insbesondere der lichtoptischen Faserplatte, einen Maximalabstand nicht überschreitet, können entgegengesetzt wirkende Elemente in der Positionierungsvorrichtung vorgesehen sein. So kann die Positionierungsvorrichtung beispielsweise mindestens ein Andruckelement umfassen, welches den medizinischen Verbrauchsartikel mit einer in Richtung des Codelesers wirkenden Kraft beaufschlagt. Beispielsweise kann das Andruckelement ausgestaltet sein, um den Verbrauchsartikel gegen den oben beschriebenen Abstandshalter zu drücken. Alternativ oder zusätzlich ist auch eine umgekehrte Kraftbeaufschlagung möglich, also eine Kraftbeaufschlagung, bei welcher das Andruckelement bewirkt, dass der Codeleser ganz oder teilweise gegen den medizinischen Verbrauchsartikel gedrückt wird. So können beispielsweise die lichtoptische Faserplatte und/oder eine die lichtoptische Faserplatte und den Bildsensor umfassende Einheit durch das Andruckelement gegen den Verbrauchsartikel gedrückt werden. Allgemein kann das Andruckelement beispielsweise ein oder mehrere Federelemente, ein oder mehrere Elemente elastischer Materialien oder ähnliche Elemente umfassen, welche üblicherweise für eine Kraftbeaufschlagung verwendet werden.

Die Positionierungsvorrichtung kann beispielsweise Bestandteil des medizinischen Geräts sein. Die Positionierungsvorrichtung kann an die Art des medizinischen Geräts und/oder die Art des medizinischen Verbrauchsartikels angepasst sein und verschiedene Arten mechanischer Vorrichtungen umfassen. Beispielsweise kann die Positionierungsvorrichtung Haltevorrichtungen umfassen, um den medizinischen Verbrauchsartikel an und/oder in dem medizinischen Gerät derart zu haltern, dass der medizinische Verbrauchsartikel in der Lesestellung, unter Wahrung der oben beschriebenen Bedingung, positioniert ist. Beispielsweise kann die Positionierungsvorrichtung dementsprechend mindestens eine Schiene zum Einführen des medizinischen Verbrauchsartikels, beispielsweise eines Testelements, umfassen. Die Positionierung kann auch eine laterale Positionierung, also beispielsweise eine Positionierung in einer Ebene senkrecht zur Verbindung zwischen Bildsensor und Verbrauchsartikel sein. Alternativ oder zusätzlich kann die Positionierungsvorrichtung auch eine Auflagefläche beinhalten, auf welcher oder an welcher der medizinische Verbrauchsartikel aufgelegt oder angelegt werden kann, so dass der Verbrauchsartikel in der Lesestellung positioniert ist.

Der Codeleser kann derart eingerichtet sein, dass der optische Code vollständig in einer aktiven Sensoroberfläche des Bildsensors abgebildet wird. Dies kann, insbesondere wenn die lichtoptische Faserplatte keine vergrößernde oder verkleinernde Wirkung aufweist, beispielsweise dadurch erfolgen, dass die aktive Sensoroberfläche des Bildsensors mindestens genauso groß ist wie die Fläche des optischen Codes. Alternativ oder zusätzlich kann die lichtoptische Faserplatte jedoch auch eine vergrößernde oder verkleinernde Wirkung aufweisen, so dass das Bild des optischen Codes, welches die optische Faserplatte auf der dem Bildsensor zuweisenden Seite generiert, größer oder kleiner ist als der eigentliche optische Code.

Alternativ kann auch lediglich ein Teil des Bilds des optischen Codes auf dem Bildsensor abgebildet werden. Beispielsweise kann wiederum in diesem Fall der Bildsensor mit seiner aktiven Fläche kleiner sein als der optische Code, oder es kann eine vergrößernde oder verkleinernde Wirkung durch die lichtoptische Faserplatte erfolgen, welche dies bewirkt. In diesem Fall nimmt der Bildsensor lediglich einen Teilbereich des optischen Codes auf. Der optische Code kann sich wiederholende, zumindest teilweise redundante Informationen umfassen. Beispielsweise kann der optische Code sich wiederholende Bitmuster bzw. Muster von Modulen umfassen, wobei diese sich wiederholenden Bitmuster bzw. Muster von Modulen derart eingerichtet sein können, dass mindestens eines dieser Bitmuster bzw. Modulmuster vollständig auf die aktive Sensoroberfläche des Bildsensors abgebildet wird.

Weitere bevorzugte Weiterbildungen der Erfindung betreffen die Ausgestaltung des medizinischen Verbrauchsartikels, zumindest im Bereich des Codes. So kann der Code und/oder die optisch lesbaren Module des Codes insbesondere durch eines oder mehrere der folgenden Verfahren auf den medizinischen Verbrauchsartikel aufgebracht werden: ein Druckverfahren auf eine Oberfläche des medizinischen Verbrauchsartikels; ein laserinduziertes Farbstoffumwandlungsverfahren; eine mechanische Deformation einer Oberfläche des medizinischen Verbrauchsartikels oder des medizinischen Verbrauchsartikels selbst. Auch andere Verfahren sind jedoch grundsätzlich einsetzbar, wie beispielsweise mechanische Ablationsverfahren, Ablation auch durch Laserbearbeitung, Implantierverfahren, Photolithographie oder ähnliche Verfahren.

Der medizinische Verbrauchsartikel kann im Bereich des optischen Codes ein Trägermaterial umfassen, welches transparente und/oder lichtstreuende Eigenschaften aufweist. Dies bedeutet, dass das Trägermaterial insbesondere für Licht mindestens einer Lichtquelle der Beleuchtungseinrichtung zumindest teilweise transparent bzw. lichtstreuend sein sollte.

Der optische Code selbst, beispielsweise die Module des optischen Codes, können auch ganz oder teilweise eingerichtet sein, um mit Licht unterschiedlicher Wellenlängen unterschiedlich zu wechselwirken. Insbesondere können dementsprechend Materialien für den optischen Code bzw. die Module verwendet werden, welche beispielsweise unterschiedliche Anregungswellenlängen, unterschiedliche Absorptionseigenschaften, unterschiedliche Streueigenschaften oder auf sonstige Weise unterschiedliche optische Eigenschaften für Anregungslicht der Beleuchtungseinrichtung aufweisen.

Der optische Code kann auf verschiedene Weise mit Licht wechselwirken. So kann der optische Code beispielsweise zumindest teilweise lumineszierende Eigenschaften aufweisen, also beispielsweise phosphoreszierende und/oder fluoreszierende Eigenschaften. Hierfür kann der optische Code, insbesondere die Module des optischen Codes, vorzugsweise entsprechende Farbstoffe, Pigmente, Phosphore oder Ähnliches aufweisen. Alternativ oder zusätzlich kann der optische Code, insbesondere die Module des optischen Codes, mindestens einen Lichtkonverter umfassen, welcher eingerichtet ist, um ein Anregungslicht, beispielsweise ein Anregungslicht der Beleuchtungseinrichtung und/oder Umgebungslicht, in ein Licht mit einer von dem Anregungslicht verschiedenen Wellenlänge umzuwandeln. In anderen Worten kann der Lichtkonverter beispielsweise einen Up-Konverter oder einen Down-Konverter umfassen, also einen Konverter, welcher Licht in höherenergetisches Licht oder niedrigerenergetisches Licht umwandeln kann. Dieser Lichtkonverter kann beispielsweise wiederum in Form eines Farbstoffs, eines Pigments, eines Phosphors oder in ähnlicher Form vorliegen. Auf diese Weise kann beispielsweise eine Anregung des Lichtkonverters mittels eines entsprechenden Anregungslichts erfolgen, welche von dem Codeleser und/oder dem Bildsensor erfasst werden kann, um die Information des Codes auszulesen.

Weitere bevorzugte Weiterbildungen betreffen die Umgebung des Codes, also den Bereich des medizinischen Verbrauchsartikels, in welchem der optische Code aufgebracht ist. So kann der medizinische Verbrauchsartikel in diesem Bereich beispielsweise ein Trägermaterial umfassen, welches diffus lichtleitende und/oder transparente Eigenschaften aufweist, insbesondere für Anregungslicht, beispielsweise Anregungslicht der Beleuchtungseinrichtung. Insbesondere kann das Trägermaterial ein Polyester, beispielsweise Melinex, umfassen. Das Trägermaterial, insbesondere das Polyester, kann weiterhin auch dotiert sein, beispielsweise mit Titandioxid. Hierdurch kann beispielsweise ein im Wesentlichen weißer Gesamteindruck des Trägermaterials entstehen, wobei das Trägermaterial dennoch diffus lichtstreuende Eigenschaften aufweist. Auf dieses Weise kann beispielsweise eine gleichmäßige Beleuchtung des Codes sichergestellt werden.

Ähnlich zu der möglichen Ausgestaltung des Codes und/oder der Module des Codes kann auch der medizinische Verbrauchsartikel im Bereich des optischen Codes einen Träger mit einem Trägermaterial umfassen, wobei der Träger weiterhin in diesem Bereich mindestens einen Lichtkonverter umfassen kann. Dieser Lichtkonverter, bei welchem es sich wiederum um einen Up-Konverter und/oder einen Down-Konverter gemäß der obigen Beschreibung handeln kann, kann insbesondere eingerichtet sein, um Anregungslicht in ein Licht unterschiedlicher Wellenlänge umzuwandeln. Der Lichtkonverter kann beispielsweise gleichmäßig in dem Trägermaterial verteilt sein, so dass Licht mit der von dem Anregungslicht verschiedenen Wellenlänge den optischen Code im Wesentlichen gleichmäßig beleuchtet. Unter "im Wesentlichen gleichmäßig" kann dabei eine isotrope Beleuchtung verstanden werden, bei welcher die Beleuchtung des Codes von einer gleichförmigen Beleuchtung um weniger als 20% abweicht.

Weitere bevorzugte Ausgestaltungen der Erfindung betreffen die Ausgestaltung des medizinischen Geräts und/oder des medizinischen Verbrauchsartikels. Hierfür kommen insbesondere die in der Beschreibung des Standes der Technik erwähnten medizinischen Systeme als bevorzugte Ausgestaltungen in Betracht. So kann das medizinische Gerät beispielsweise ein Analysegerät zum Nachweis mindestens eines Analyten in einer Probe umfassen, beispielsweise einer flüssigen Probe, insbesondere einer Körperflüssigkeit. Als Analyt kommen insbesondere Metaboliten in der Körperflüssigkeit in Frage, beispielsweise Glukose, Cholesterin und/oder ähnliche Metaboliten. Auch beispielsweise ein Koagulationsnachweis ist möglich. Das Analysegerät kann dann eingerichtet sein, um mit einem medizinischen Verbrauchsartikel in Form mindestens eines Testelements, insbesondere einem Teststreifen und/oder einem Testband, zusammenzuwirken. Dabei kann ein einzelner Teststreifen als medizinischer Verbrauchsartikel verstanden werden, oder, wie oben ausgeführt, auch eine Mehrzahl von Teststreifen und/oder Testbändern, welche beispielsweise in einem entsprechenden Magazin oder einem Gehäuse aufgenommen sein können. In letzterem Fall können auch das Magazin und/oder das Gehäuse entsprechend mit dem optischen Code versehen sein.

Alternativ oder zusätzlich kann das medizinische System auch eine Dosiervorrichtung zur Dosierung mindestens eines Medikaments umfassen, insbesondere eine Medikationspumpe, beispielsweise eine Insulinpumpe. In diesem Fall kann der Verbrauchsartikel beispielsweise einen Katheter bzw. eine Kanüle (beide Begriffe werden in der folgenden Beschreibung synonym verwendet) umfassen, mit welchem die Dosiervorrichtung zusammenwirkt. Das medizinische System kann dementsprechend beispielsweise als so genanntes "Infusionsset" ausgestaltet sein. Da Katheter üblicherweise mit Luft befüllt ausgeliefert werden, muss vor der Applikation am und/oder im Körper eine Spülung mit einer medizinischen Flüssigkeit, wie beispielsweise mit einer Insulininfusion, durchgeführt werden, um die Luft aus dem Katheter zumindest weitgehend zu verdrängen. Das für den jeweiligen Katheter spezifische Füllvolumen kann manuell in die Dosiervorrichtung, beispielsweise die Dosierpumpe, eingegeben werden, so dass eine entsprechende Dosierung zum Durchspülen des Katheters erfolgt. Dieser Spülvorgang wird als "Priming" bezeichnet. Die manuelle Eingabe der Priming-Parameter ist jedoch, wie oben dargestellt, risikobehaftet, da diese manuelle Eingabe beispielsweise fehlerhaft oder gar nicht erfolgen kann. Zu bevorzugen ist daher ein automatisches Priming ("Auto-Priming"), wie es beispielsweise in WO 2007/128144 offenbart wird. Erfindungsgemäß kann der Codeleser daher eingerichtet sein und genutzt werden, um mindestens eine Information über ein Füllvolumen des Katheters aus dem optischen Code, welcher beispielsweise auf dem Katheter oder einer Verpackung des Katheters aufgebracht sein kann, auszulesen. Auf diese Weise lässt sich das Auto-Priming erheblich vereinfachen.

Allgemein und unabhängig von der sonstigen Ausgestaltung des medizinischen Systems kann die Information, welche von dem Codeleser aus dem optischen Code ausgelesen wird, eine Vielzahl möglicher Informationen umfassen. Beispielsweise, jedoch nicht abschließend, kann die Information eine chargenspezifische Information über den medizinischen Verbrauchsartikel umfassen. Unter einer chargenspezifischen Information ist dabei eine Information zu verstehen, welche allgemein von medizinischen Verbrauchsartikel zu medizinischem Verbrauchsartikel wechseln kann. Beispielsweise kann diese Information eine Information über eine Charge, eine Information über chargenspezifische Besonderheiten des medizinischen Verbrauchsartikels, eine Information über einen Hersteller, eine Nummer (beispielsweise eine Seriennummer), einen Produktionsparameter, eine Information über die Art und Weise, wie die Funktionalität des medizinischen Geräts auf die spezielle Charge des medizinischen Verbrauchsartikels anzupassen ist oder Ähnliches umfassen. Alternativ oder zusätzlich kann eine Chargennummer des medizinischen Verbrauchsartikels umfasst sein. Weiterhin kann, ebenfalls alternativ oder zusätzlich, mindestens ein mathematischer Parameter und/oder Parametersatz umfasst sein, welcher für ein korrektes Zusammenwirken zwischen dem medizinischen Gerät und dem medizinischen Verbrauchsartikel von dem medizinischen Gerät benötigt wird. Weiterhin kann, ebenfalls alternativ oder zusätzlich, ein Datum und/oder ein Verfallsdatum umfasst sein. Außerdem kann, ebenfalls alternativ oder zusätzlich, eine Anweisung an einen Benutzer des medizinischen Systems umfasst sein, beispielsweise eine Information darüber, welche Art medizinischer Verbrauchsartikel gerade verwendet wird und/oder wie dieser medizinische Verbrauchsartikel zu handhaben ist. Diese Anweisung kann auch beispielsweise über eine Anzeigevorrichtung des medizinischen Geräts, beispielsweise eine visuelle und/oder akustische Anzeigevorrichtung, an den Benutzer ausgegeben werden. Weiterhin kann, ebenfalls alternativ oder zusätzlich, eine Herstellerinformation umfasst sein, so dass beispielsweise mittels des medizinischen Systems auch eine Fälschungssicherung betrieben werden kann. Auf diese Weise kann beispielsweise verhindert werden, dass gefälschte medizinische Verbrauchsartikel, also medizinische Verbrauchsartikel, welche von nicht-autorisierten Herstellern stammen, verwendet werden, was zu fatalen Folgen führen kann. In diesem Fall kann das medizinische System beispielsweise wiederum eingerichtet sein, um einen Benutzer des medizinischen Systems über den Hersteller und/oder über die Tatsache, dass eine Fälschung vorliegt, zu informieren und/oder um andere geeignete Maßnahmen zu treffen, beispielsweise eine Sperrung ein oder mehrerer Funktionalitäten und/oder eine Dokumentation der Verwendung des gefälschten Verbrauchsartikels. Weiterhin kann die Information, ebenfalls alternativ oder zusätzlich, auch eine Kalibrationsinformation umfassen, wobei unter einer Kalibrationsinformation allgemein Informationen darüber zu verstehen sind, wie beispielsweise Messergebnisse, welche das medizinische Gerät mittels des medizinischen Verbrauchsartikels (beispielsweise eines Testelements) erzielt, auszuwerten sind. Auch hier können wieder chargenspezifische Unterschiede berücksichtigt sein.

Das medizinische Gerät kann insbesondere als Handgerät ausgestaltet sein, also als Gerät, welches ohne Zuhilfenahme von Transporteinrichtungen von einem Benutzer mit der Hand gehalten werden kann. In diesem Fall machen sich das geringe Gewicht und die Kompaktheit des verwendeten Codelesers besonders vorteilhaft bemerkbar. Das Handgerät kann auch mindestens einen elektrischen Energiespeicher umfassen, insbesondere eine Batterie und/oder einen Akkumulator.

Entsprechend der Art des medizinischen Systems kann der medizinische Verbrauchsartikel auf viele verschiedene Weisen ausgestaltet sein. Es können auch mehrere unterschiedliche Arten von medizinischen Verbrauchsartikeln mit dem medizinischen System zusammenwirken, wobei unterschiedliche Codeleser für die optischen Codes der unterschiedlichen Verbrauchsartikel eingesetzt werden können, oder wobei auch ein und derselbe Codeleser für die unterschiedlichen Arten medizinischer Verbrauchsartikel verwendet werden kann.

So umfasst das medizinische System mindestens einen medizinischen Verbrauchsartikel, welcher mindestens einen von dem Codeleser auslesbaren optischen Code aufweist, wobei der medizinische Verbrauchsartikel beispielsweise einen oder mehrere der folgenden Verbrauchsartikel umfassen kann: ein Testelement zum Nachweis mindestens eines Analyten in einer Probe, insbesondere einen Teststreifen oder ein Testband; ein Magazin zur Aufnahme mindestens eines Testelements zum Nachweis mindestens eines Analyten in einer Probe, insbesondere eines Teststreifen oder eines Testbands; eine Lanzette zur Erzeugung einer Öffnung in einer Hautoberfläche eines Patienten; ein Magazin zur Aufnahme mindestens einer Lanzette zur Erzeugung einer Öffnung in einer Hautoberfläche eines Patienten; eine Medikamentenverpackung, insbesondere eine Insulinkartusche; einen Katheter und/oder eine Kanüle.

So kann der medizinische Verbrauchsartikel beispielsweise ein Testelement, insbesondere einen Teststreifen und/oder ein Testband und/oder ein Testrad und/oder ein faltbares Testelement, zum Nachweis mindestens eines Analyten in einer Probe umfassen. Auf die obige Beschreibung des Standes der Technik kann beispielhaft verwiesen werden. Das Testelement kann, wie oben dargelegt, beispielsweise mittels eines optischen und/oder eines elektrochemischen Tests einen quantitativen und/oder qualitativen Nachweis des Analyten führen. Alternativ oder zusätzlich zu einzelnen Testelementen können auch Magazine zur Aufnahme mindestens eines derartigen Testelements vorgesehen sein, wobei auch die Magazine als Verbrauchsartikel codiert sein können.

Alternativ oder zusätzlich zu Testelementen kann der medizinische Verbrauchsartikel beispielsweise auch eine Lanzette zur Erzeugung einer Öffnung in einer Hautoberfläche eines Patienten umfassen oder ein Magazin zur Aufnahme mindestens einer derartigen Lanzette. Weiterhin kann der medizinische Verbrauchsartikel eine Medikamentenverpackung, insbesondere eine Verpackung für flüssige Medikamente, umfassen. Beispielsweise kann diese Medikamentenverpackung eine Insulinkartusche umfassen. Weiterhin kann, wie oben dargelegt, der medizinische Verbrauchsartikel beispielsweise einen Katheter umfassen.

Das medizinische System gemäß einer oder mehrerer der oben beschriebenen vorteilhaften Ausführungsformen weist eine Vielzahl von Vorteilen gegenüber bekannten derartigen medizinischen Systemen auf. Bereits genannt wurden insbesondere die Kompaktheit und die Ausgestaltung des Systems mit einem geringen Gewicht, welches sich insbesondere bei Handgeräten vorteilhaft bemerkbar macht. So kann beispielsweise für den Codeleser eine Baugröße von 1 Kubikzentimeter oder weniger erreicht werden.

Weiterhin ist die erfindungsgemäße Lösung auch für eine große Informationstiefe geeignet. Optimal ist die Eignung beispielsweise für Informationen von 20 bis 100 bit. Beispielsweise können Informationen von 40 bit Nutzinformation und 40 bit Kontroll- und Redundanzinformation, also insgesamt 80 bit, verwendet werden. Die Fläche des optischen Codes kann dabei beispielsweise 10 bis 100 mm² betragen. Damit lassen sich einerseits, wie oben erwähnt, Codeleser kleiner Bauart realisieren bzw. einsetzen. Weiterhin werden dadurch die Anforderungen an die Technik, mit welcher der optische Code aufgebracht wird, beispielsweise an die Drucktechnik und/oder die anderen oben genannten Aufbringmethoden, verringert, was beispielsweise auch eine kostengünstigere Herstellung ermöglicht.

Die Beleuchtung des optischen Codes kann auf sehr kurzem Wege erfolgen und dennoch eine hinreichende Homogenität der Beleuchtung sicherstellen. Auf diese Weise kann ein sicheres Auslesen des Codes gewährleistet sein, was insgesamt zur Betriebs- und Handhabungssicherheit des medizinischen Systems beiträgt. Das medizinische Gerät kann eine oder mehrere Steuerungen, beispielsweise ein oder mehrere Datenverarbeitungsgeräte, umfassen, welche eine Umwandlung eines von dem Bildsensor aufgenommenen Bildes des vollständigen oder teilweisen optischen Codes in die entsprechende Information umwandeln, so dass diese Information des optischen Codes ausgelesen werden kann. Die Steuerung kann in dem Codeleser integriert sein, oder kann auch ganz oder teilweise mit sonstigen Steuerungen des medizinischen Geräts zusammengefasst werden, beispielsweise mit einer ohnehin in vielen medizinischen Geräten, wie beispielsweise Glukosemessgeräten, vorhandenen zentralen Steuerung. Insbesondere können Beleuchtungs- und Auswertefunktionen auch in ein einziges Modul integriert werden, beispielsweise ein einziges Modul des Codelesers. Dieses einzige Modul kann dann sehr nahe an den medizinischen Verbrauchsartikel, insbesondere den optischen Code desselben, herangeführt werden, so dass nicht nur der Codeleser selbst sondern auch der Codeleser einschließlich des optischen Codes auf dem medizinischen Verbrauchsartikel einen vergleichsweise kleinen Bauraum einnimmt, beispielsweise einen Bauraum von ca. 1 Kubikzentimeter.

Der Codeleser kommt vorzugsweise vollständig ohne abbildende Systeme aus, d.h. ohne Linsen-, insbesondere Mikrolinsensysteme, oder ohne gekrümmte Spiegel oder ähnliches. Auf derartige abbildende Systeme lässt sich durch die Verwendung der lichtoptischen Faserplatte vollständig verzichten, da mittels der lichtoptischen Faserplatte der Bildsensor vorzugsweise direkt virtuell auf den optischen Code aufgesetzt werden kann. Auch dies trägt erheblich zu einer Verringerung des Bauraums bei.

Die Möglichkeit der oben beschriebenen zeitaufgelösten Messung, beispielsweise durch ein Abfrage-Antwort-Pulsschema, ermöglicht eine Messung auch bei ungünstigen Lichtverhältnissen. So kann beispielsweise mittels dieses Pulsschemas durch einen Impuls und die Messung des nachleuchtenden Lichts das Verhältnis von Nutzsignal und Störsignalverbessert werden, da beispielsweise die Problematik eines Streulicht-Untergrunds verringert wird. Hierdurch wird die Signalqualität und die Zuverlässigkeit der ausgelesenen Information verbessert.

Weiterhin ist das medizinische System weitgehend indifferent gegenüber der Art der codierten Information. Wie oben dargestellt, kann diese Information beispielsweise eine chargenspezifische Information umfassen. Die codierte Information kann beispielsweise binär codiert sein, insbesondere zweidimensional. Durch die hohe Ortsauflösung kann eine hohe Speicherdichte der Information in dem optischen Code gewährleistet sein.

Die Auswertung des optischen Codes mittels des Codelesers kann, wie oben dargestellt, auch auf komplexe Messschemata zurückgreifen. So können, wie dargelegt, beispielsweise zeitaufgelöste Messschemata verwendet werden. Alternativ oder zusätzlich können wellenlängenselektive Messschemata verwendet werden, beispielsweise unter Verwendung mehrerer Anregungswellenlängen, unter Verwendung mehrerer Anregungswellenlängen, unter Verwendung mehrerer vom optischen Code bzw. den Modulen kommenden Antwortwellenlängen oder durch Kombinationen dieser Verfahren. Alternativ oder zusätzlich lässt sich der Code auch durch eine mechanische Ausgestaltung erzielen, beispielsweise durch eine flache, deformierte, hohle oder ähnliche mechanische Ausgestaltung einer Oberfläche des medizinischen Verbrauchsartikels im Bereich des optischen Codes.

Weiterhin weist das medizinische System, insbesondere der Codeleser dieses medizinischen Systems, eine hohe Flexibilität hinsichtlich der Beleuchtung des optischen Codes auf. Wie oben dargestellt, kann zu dieser Beleuchtung beispielsweise eine Beleuchtungseinrichtung verwendet werden. Alternativ oder zusätzlich können auch anderweitig vorhandene Lichtquellen genutzt werden, beispielsweise Umgebungslicht. Die Beleuchtung kann beispielsweise ganz oder teilweise durch den medizinischen Verbrauchsartikel hindurch, auf diffuse Weise innerhalb des Verbrauchsartikels, diffus durch die lichtoptische Faserplatte, von der Seite der lichtoptischen Faserplatte, durch die lichtoptische Faserplatte hindurch, durch den Bildsensor hindurch oder mittels einer Kombination der genannten Beleuchtungsarten oder anderer Beleuchtungsarten erfolgen.

Oben wurde in einer Ausführungsform des medizinischen Systems beschrieben, dass eine Beleuchtung auch durch den Bildsensor hindurch erfolgen kann. Wie dem Fachmann unmittelbar klar ist, lässt sich diese Ausgestaltung einer Beleuchtung durch den Bildsensor hindurch auch auf andere medizinische Systeme oder andere medizinische Geräte übertragen, bei welchen eine Kombination eines Bildsensors mit einer Beleuchtungseinrichtung vorgesehen ist. Diese medizinischen Systeme beziehungsweise medizinischen Geräte müssen dabei nicht notwendigerweise einen Codeleser oder eine lichtoptische Faserplatte umfassen.

Neben medizinischen Systemen und medizinischen Geräten mit Codelesern lassen sich hier exemplarisch medizinische Geräte nennen, bei welchen mittels des Bildsensors beispielsweise ein Testelement ausgewertet wird. Beispielsweise kann dieses Testelement mindestens ein Testfeld umfassen, mit mindestens einer Testchemie, welche für einen qualitativen und/oder quantitativen Nachweis mindestens eines Analyten in einer Probe, beispielsweise einer Körperflüssigkeit, geeignet ist. Derartige Testelemente sind beispielsweise als Teststreifen aus dem Stand der Technik hinlänglich bekannt. Auch diese können mit einem oder mehreren Bildsensoren ausgewertet werden. Beispielsweise kann das mindestens eine Testfeld eine Farbe oder eine sonstige optische nachweisbare Eigenschaft bei Anwesenheit des mindestens einen Analyten ändern. Auch in diesem oder in anderen Fällen ist dabei in der Regel also eine Beleuchtung des von dem Bildsensor betrachteten Gegenstands, sei es nun ein Barcode, ein Testfeld oder eine andere Art von Gegenstand, erforderlich. Ohne Beschränkung weiterer möglicher Arten der Wechselwirkung des Lichts mit dem Gegenstand, welche beispielsweise reflektiver Art, transmissiver Art oder anregender Art sein kann, wird das Licht, mit welchem der Gegenstand beaufschlagt wird, im folgenden als Anregungslicht bezeichnet.

In einem weiteren Aspekt wird daher ein medizinisches Gerät zur Durchführung mindestens einer medizinischen Funktion vorgeschlagen. Für die mögliche Ausgestaltung dieser medizinischen Funktion oder die Ausgestaltung des medizinischen Geräts kann exemplarisch auf die obige Beschreibung verwiesen werden. Insbesondere kann er das medizinische Gerät in einem medizinischen System gemäß einer oder mehreren der oben beschriebenen Ausgestaltungen eingesetzt werden. Auch andere Einsatzgebiete sind jedoch grundsätzlich möglich.

Das medizinische Gerät umfasst mindestens einen Bildsensor mit einer Mehrzahl von Sensoren. Weiterhin umfasst das medizinische Gerät mindestens eine Beleuchtungseinrichtung, welche ihrerseits mindestens eine Lichtquelle aufweist. Die Lichtquelle ist eingerichtet, um mindestens einen Gegenstand, insbesondere einen medizinischen Verbrauchsartikel, durch den Bildsensor hindurch zu beleuchten.

Wie oben dargestellt, kann der mindestens eine Gegenstand auf unterschiedliche Weisen ausgestaltet sein. Beispielsweise kann es sich dabei um einen medizinischen Verbrauchsartikel, insbesondere einen medizinischen Verbrauchsartikel mit mindestens einem Barcode, handeln. Alternativ oder zusätzlich kann es sich bei dem Gegenstand jedoch auch um mindestens ein Testelement mit mindestens einem Testfeld handeln, welches zum Nachweis mindestens eines Analyten in einer Probe, insbesondere zum Nachweis mindestens eines Analyten in einer Körperflüssigkeit, eingerichtet ist. Verschiedene Ausgestaltungen sind möglich.

Die Beleuchtung des Gegenstands durch den Bildsensor hindurch kann auf unterschiedliche Weisen realisiert werden, wobei wiederum exemplarisch auf die obige Beschreibung des medizinischen Systems und dort insbesondere auf die Beschreibung des Bildsensors und/oder der Beleuchtungseinrichtung verwiesen werden kann. Auch andere Ausgestaltungen sind jedoch möglich. So kann beispielsweise der Bildsensor selbst zumindest teilweise transparent sein für ein Anregungslicht der Lichtquelle. Der Bildsensor kann eine Mehrzahl von Öffnungen aufweisen, durch welche Anregungslicht der Lichtquelle hindurch treten kann. Alternativ oder zusätzlich kann der Bildsensor ein Material aufweisen, welches zumindest teilweise transparent für Anregungslicht der Lichtquelle ist. Der Bildsensor kann insbesondere ein Halbleitermaterial mit einer Bandlücke aufweisen, wobei die Lichtquelle eingerichtet ist, um Licht mit einer geringeren Energie als die Bandlücke zu emittieren.

Unabhängig von der Verwendung der lichtoptischen Faserplatte bietet also die Beleuchtung des Gegenstands durch den Bildsensor hindurch die Möglichkeit, einerseits eine ausreichende und vorzugsweise möglichst homogene Beleuchtung des Gegenstands oder zumindest eines relevanten Bereichs des Gegenstands, beispielsweise eines Testfelds, zu gewährleisten.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: ein erstes Ausführungsbeispiel eines erfindungsgemäßen medizinischen Systems mit einem Blutglukosemessgerät;
- Figur 2: ein zweites Ausführungsbeispiel eines medizinischen Systems mit einer Insulinpumpe und einem Infusionsset;
- Figur 3: einen schematischen Aufbau eines herkömmlichen Barcodelesers;
- Figur 4: ein erstes Ausführungsbeispiel eines erfindungsgemäßen Codelesers mit einer Durchlicht-Beleuchtung;

- Figuren 5A und 5B: verschiedene Detaildarstellungen der in Figur 4 verwendeten lichtoptischen Faserplatte;
- Figur 6: ein zu Figur 4 alternatives zweites Ausführungsbeispiel eines Codelesers mit einer Lichteinkopplung in die lichtoptische Faserplatte von der Seite;
- Figur 7: ein Ausführungsbeispiel des Codelesers mit Lichteinkopplung in einen Teststreifen bei absorbierendem optischem Code;
- Figur 8: ein Ausführungsbeispiel eines Codelesers mit Lichteinkopplung in einen Teststreifen bei fluoreszierendem optischem Code;
- Figuren 9A und 9B: Ausführungsbeispiele eines Codelesers mit Lichteinkopplung durch einen Bildsensor hindurch;
- Figur 10: ein Ausführungsbeispiel eines redundanten optischen Codes;
- Figur 11: ein Ausführungsbeispiel eines möglichen zeitaufgelösten Messschemas; und
- Figur 12: ein Ausführungsbeispiel eines Codelesers mit einer Positionierungsvorrichtung.

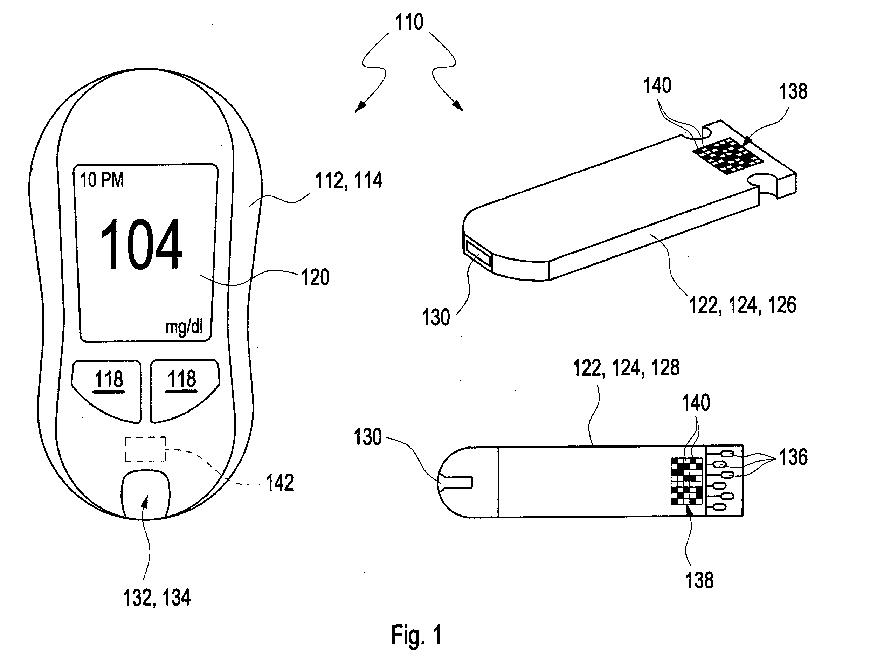

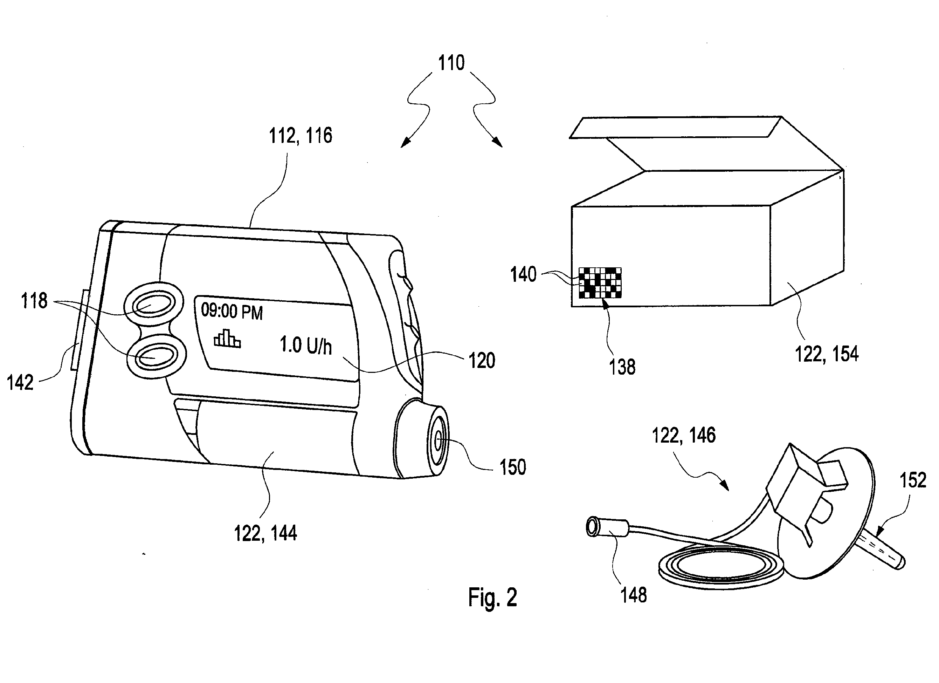

In den Figuren 1 und 2 sind beispielhaft zwei verschiedene Ausführungen medizinischer Systeme 110 gemäß der vorliegenden Erfindung dargestellt. Diese medizinischen Systeme 110 umfassen jeweils ein medizinisches Gerät 112, welches der eigentliche Funktionsträger einer Hauptfunktion des medizinischen Systems 110 ist. Dabei ist beispielhaft bei dem Ausführungsbeispiel in Figur 1 das medizinische Gerät 112 als Blutzuckermessgerät 114 ausgestaltet, wohingegen das medizinische Gerät 112 in dem Ausführungsbeispiel gemäß Figur 2 als Insulinpumpe 116 ausgestaltet ist. Das medizinische Gerät 112 kann insbesondere ausgestaltet sein, um eine Interaktion mit einem Benutzer zu ermöglichen. Beispielsweise kann über Eingabemittel 118 einem Benutzer die Möglichkeit einer Bedienung mechanischer und/oder elektrischer Funktionen des medizinischen Geräts 112 ermöglicht werden. Weiterhin können Ausgabemittel 120 vorgesehen sein, beispielsweise ein oder mehrere Displays, welche beispielsweise Messwerte, eingestellte Parameter oder sonstige Informationen an einen Benutzer ermöglichen. Das medizinische Gerät 112 kann also die eigentliche "Schnittstelle" des medizinischen Systems 110 zum Benutzer darstellen.

Daneben umfasst das medizinische System 110 ein oder mehrere medizinische Verbrauchsartikel 122. Bei dem in Figur 1 dargestellten medizinischen System wirkt das Blutzuckermessgerät 114 beispielsweise mit Testelementen 124 zusammen, welche beispielhaft in Figur 1 in zwei Ausführungsformen gezeigt sind. Während die obere, mit der Bezugsziffer 126 bezeichnete Ausführungsform ein steifes Testelement mit flacher, länglicher Gestalt zeigt, zeigt die untere Ausführungsform in Figur 1 einen Teststreifen 128. Das in Figur 1 dargestellte Blutzuckermessgerät 114 ist in der Regel für die Verwendung von Teststreifen 128 ausgelegt, wobei jedoch auch andere Arten von Testelementen, beispielsweise das Testelement 126, verwendet werden können.

Beide Testelemente 124 weisen eine Applikationsstelle 130 auf, an welcher eine Probe einer Körperflüssigkeit auf das Testelement 124 aufgebracht werden kann. Dies kann bei in das Blutzuckermessgerät 114 eingegebenem Testelement 124 erfolgen oder, in verschiedenen Systemen auch außerhalb des Blutzuckermessgeräts. Das Blutzuckermessgerät 114 weist eine Eingabeöffnung 132 auf, welche gleichzeitig als Positioniervorrichtung 134 dient, um bei korrekt in die Eingabeöffnung 132 eingeschobenem Testelement 124 ein Zusammenwirken des Blutzuckermessgeräts 114 mit dem Testelement 124 zu ermöglichen.

Die Auswertung der auf die Applikationsstelle 130 aufgebrachten Probe kann beispielsweise auf optischem oder elektrochemischem Wege erfolgen. Bei dem Teststreifen 128 sind zu diesem Zweck beispielsweise Elektrodenkontakte 136 vorgesehen, welche bei in das Blutzuckermessgerät 114 eingeschobenem Teststreifen 128 von dem Blutzuckermessgerät 114 kontaktiert werden.

Da sich die Testelemente 124 von Charge zu Charge ändern können, wird vorgeschlagen, auf den Testelementen 124 einen optischen Code 138 aufzubringen. Dieser optische Code 138 ist in den dargestellten Ausführungsbeispielen lediglich symbolisch angedeutet und kann beispielsweise als zweidimensionaler Barcode ausgestaltet sein. Beispielsweise kann der Code ein 35-bit-Code sein, welcher sich aus 5 x 7 kleinsten Einheiten zusammensetzt. Diese kleinsten Einheiten werden auch als Module 140 bezeichnet. In diesen kleinsten Einheiten, welche im dargestellten Ausführungsbeispiel beispielsweise "weiß" oder "schwarz" sein können, enthalten die eigentliche Information in beispielsweise binärer Form. Auch alternative Ausgestaltungen sind möglich, beispielsweise durch eine Verwendung von Zwischenstufen zwischen Schwarz und Weiß, also von Graustufen- bzw. entsprechenden Farbabstufungen. Die Begriffe Schwarz, Weiß, Grau und Farbe gelten sinnentsprechend im gesamten Wellenlängenbereich von 300-3000 nm.

Als Gegenstück zu dem optischen Code 138 umfasst das medizinische Gerät 112 in Form des Blutzuckermessgeräts 114 einen Codeleser 142, welcher in Figur 1 lediglich angedeutet ist. Dieser Codeleser 142 kann beispielsweise in der Nähe der Eingabeöffnung 132 innerhalb eines Gehäuses des Blutzuckermessgeräts 114 angeordnet sein, so dass in einer festen Position bei in die Positioniervorrichtung 134 eingeschobenem Testelement 124 (statische Messung) oder während des Einschiebens des Testelements (dynamisch) der optische Code 138 von dem Codeleser 142 ausgelesen werden kann. Beispielsweise kann dieser Codeleser 142 ein Codeleser für 5 x 7 Pixel sein. Ausführungsbeispiele des Codelesers werden nachfolgend näher beschrieben:

Bei dem Ausführungsbeispiel gemäß Figur 1 ist das Testelement 124 unmittelbar als medizinischer Verbrauchsartikel 122 anzusehen, auf welchem der optische Code 138 aufgebracht ist. Alternativ oder zusätzlich kann der optische Code 138 jedoch auch beispielsweise auf einem medizinischen Verbrauchsartikel 122 in Form einer Verpackung der Testelemente 124 angeordnet sein. In diesem Fall kann der Codeleser 142 beispielsweise ganz oder teilweise als ein auf einer Außenseite eines Gehäuses des Blutzuckermessgeräts 114 angebrachter Codeleser ausgestaltet sein, welcher beispielsweise eingerichtet ist, um den optischen Code 138 auf dieser Verpackung auszulesen.

Bei dem in Figur 2 gezeigten medizinischen System sind beispielhaft drei verschiedene Ausführungsformen medizinischer Verbrauchsartikel 122 dargestellt. So besteht zum einen die Möglichkeit, eine Insulinkartusche 144 als medizinischen Verbrauchsartikel 122 auszugestalten. Auch in diesem Fall kann ein optischer Code 138 (in Figur 2 nicht dargestellt) auf dieser Insulinkartusche 144 aufgebracht werden. Alternativ oder zusätzlich kann jedoch auch eine Primärkartusche für einen Insulinvorrat verwendet werden, wobei Insulin von dieser Primärkartusche in die Insulinkartusche 144 umgefüllt wird. In diesem Fall kann beispielsweise die Primärkartusche, aus welcher Insulin in die Insulinkartusche 144 umgefüllt wird, selbst oder eine Verpackung derselben mit einem optischen Code 138 codiert sein und somit als medizinischer Verbrauchsartikel 122 wirken.

Daneben ist in Figur 2 ein medizinischer Verbrauchsartikel 122 in Form eines Infusionssets 146 dargestellt. Dieses Infusionsset 146 enthält eine Schlauchkanüle 148, welche an einen Adapter 150 der Insulinpumpe 116 anschließbar ist, sowie die eigentliche Kanüle 152 zum Einführen in ein Körpergewebe. Wie oben dargelegt, stellt das Füllvolumen des gesamten Infusionssets 146 oder von Teilen davon einen wesentlichen Parameter dar, welcher für das "Priming" von der Insulinpumpe 116 benötigt wird. Zu diesem Zweck kann beispielsweise auf dem Infusionsset 146 selbst oder auf einer Verpackung 154 desselben, welche ebenfalls als medizinischer Verbrauchsartikel 122 angesehen werden kann, wiederum ein optischer Code 138 vorgesehen sein. Dieser optische Code kann wiederum mittels eines Codelesers 142 von der Insulinpumpe 116 eingelesen werden, so dass die Insulinpumpe 116 diese Informationen über das Füllvolumen für einen Priming-Vorgang nutzen kann. Auch andere Arten von Informationen können auf diese Weise übertragen werden. Der Codeleser 142 ist in Figur 2 symbolisch an einem Ende eines Gehäuses der Insulinpumpe 116 angeordnet und kann beispielsweise zum Auslesen der Information auf den optischen Code 138 auf der Verpackung 154 und/oder dem Infusionsset 146 aufgesetzt werden.

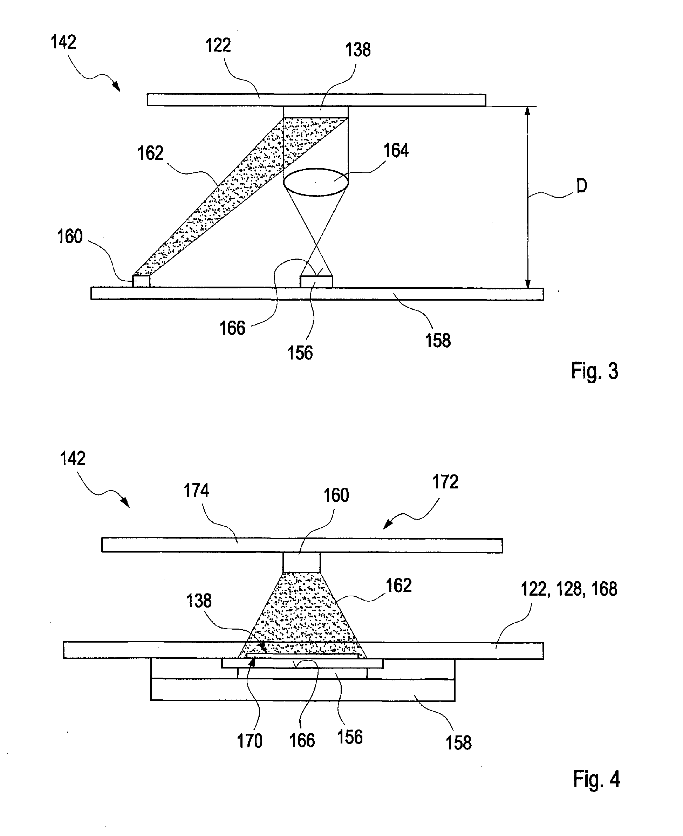

In Figur 3 ist ein dem Stand der Technik entsprechendes Beispiel eines Codelesers 142 schematisch dargestellt. Der Codeleser 142 umfasst einen Bildsensor 156 mit einer Mehrzahl einzelner Sensoren, welche in Figur 3 nicht aufgelöst dargestellt sind. Beispielsweise können diese einzelnen Sensoren eindimensional oder zweidimensional angeordnet sein. Der Bildsensor kann beispielsweise als Array von Photodioden, als CCD-Chip, als CMOS-Chip, als organischer Photodetektor (OPD) oder auf ähnliche Weise ausgestaltet sein. Der Bildsensor 156 kann insbesondere auf einer Sensorplatine 158 angeordnet sein.

Darüber hinaus umfasst der Codeleser 142 gemäß dem Stand der Technik eine Lichtquelle 160, beispielsweise eine oder mehrere Leuchtdioden. Diese Lichtquelle 160 dient dazu, den optischen Code 138 auf dem medizinischen Verbrauchsartikel 122 mit einem Beleuchtungslicht 162 zu beleuchten. Je nach Art der Wechselwirkung dieses Beleuchtungslichts 162 mit dem optischen Code 138 kann dieses Beleuchtungslicht auch als Anregungslicht fungieren, wobei, unabhängig von der Art der Wechselwirkung, beide Begriffe im Rahmen der vorliegenden Erfindung synonym verwendet werden.

Der derart beleuchtete optische Code 138 wird über ein Abbildungssystem 164, welches in Figur 3 symbolisch in Form einer einzelnen Linse dargestellt ist, auf eine aktive Sensoroberfläche 166 des Bildsensors 156 abgebildet. Diese Abbildung erfolgt über abbildende optische Elemente, wie beispielsweise Linsen oder Linsensysteme.

Bei den dem Stand der Technik entsprechenden Codelesern 142 gemäß Figur 3 ist ein erheblicher Abstand (in Figur 3 mit D bezeichnet) zwischen dem Codeleser 142 bzw. der Elektronikplatine 158 und dem medizinischen Verbrauchsartikel 122 erforderlich. Dies ist zum einen dadurch bedingt, dass das Beleuchtungslicht 162 seitlich auf die Oberfläche des optischen Codes 138 auftreffen muss, wobei diese gleichmäßig beleuchtet sein muss, und wobei das Beleuchtungslicht 162 nicht durch das Abbildungssystem 164 behindert werden darf. Weiterhin wird der Mindestabstand, welcher in der Regel etliche Millimeter beträgt, durch das Abbildungssystem 164 mitbedingt, da dieses den optischen Abbildungsgesetzen genügen muss. Zudem treibt das Erfordernis des Abbildungssystems 164 die Kosten für den Codeleser 142 erheblich in die Höhe. Insgesamt ist also ein Codeleser 142 gemäß Figur 3 aus Kostenaspekten und aus Bauraumaspekten für tragbare medizinische Geräte 142, beispielsweise gemäß den Figuren 1 oder 2, mit einigen Nachteilen behaftet.

In Figur 4 wird hingegen ein erstes Ausführungsbeispiel eines erfindungsgemäßen Codelesers 142 gezeigt. Bei diesem Codeleser wird wiederum ein medizinischer Verbrauchsartikel 122 verwendet, bei welchem im folgenden und ohne Beschränkung möglicher weiterer Ausführungsformen, angenommen wird, dass es sich um einen Teststreifen 128 handelt.

Der Teststreifen 128 umfasst in diesem Ausführungsbeispiel ein Trägermaterial 168, auf welches der optische Code 138 aufgebracht ist. Im Gegensatz zu dem Ausführungsbeispiel gemäß Figur 3 wird jedoch bei der erfindungsgemäßen Ausgestaltung gemäß Figur 4 vorzugsweise keinerlei Abbildungssystem 164 verwendet, sondern der optische Code 138 sitzt auf einer lichtoptischen Faserplatte 170 auf oder ist unmittelbar vor dieser Faserplatte 170 positioniert. Diese lichtoptische Faserplatte 170 wiederum ist unmittelbar über dem Bildsensor 156 angeordnet oder sitzt auf diesem Bildsensor 156 auf, wobei jedoch optional auch eine oder mehrere Zwischenschichten vorgesehen sein können. Der Bildsensor 156 ist wiederum beispielsweise auf einer Sensorplatine 158 angeordnet und kann beispielsweise ausgestaltet sein wie in Figur 3 beschrieben.

Das Trägermaterial 168 des Teststreifens 128 ist ganz oder teilweise aus einem zumindest teilweise für das Beleuchtungslicht 162 transparenten Material hergestellt. Die Lichtquelle 160, welche Bestandteil einer Beleuchtungseinrichtung 172 mit einer optionalen Beleuchtungsplatine 174 ist, ist in diesem Ausführungsbeispiel auf der Rückseite des Teststreifens 128, also auf der dem optischen Code 138 abgewandten Seite des Teststreifens 128, angeordnet. Das Beleuchtungslicht 162 durchdringt das Trägermaterial 168 und wechselwirkt mit dem optischen Code 138. Dies kann auf verschiedene Weisen erfolgen. Beispielsweise kann das Beleuchtungslicht 162 durch das Material des optischen Codes 138 absorbiert werden, so dass ein Schattenbild entsteht. Alternativ oder zusätzlich kann das Material des optischen Codes 138 jedoch auch lumineszierend, beispielsweise fluoreszierend, ausgestaltet sein und den optischen Code zur Emission von Lumineszenzlicht anregen. Wiederum alternativ oder zusätzlich kann das Beleuchtungslicht 162 auch mit dem Trägermaterial 168 wechselwirken, beispielsweise einem in diesem Trägermaterial 168 aufgenommenen Lumineszenzkonverter oder Farbstoff, welcher ein sekundäres Beleuchtungslicht 162 erzeugt, das wiederum den optischen Code 138 von hinten beleuchtet. Letzteres kann beispielsweise zu einer Vergleichmäßigung des Beleuchtungslichts 162 verwendet werden.

In jedem der dargestellten Fälle entsteht an der Oberfläche des optischen Codes 138 ein Bild des optischen Codes, welches wahrnehmbar ist. Dieses Bild des optischen Codes 138 wird durch die lichtoptische Faserplatte 170 zu der aktiven Sensoroberfläche 166 des Bildsensors 156 geleitet.

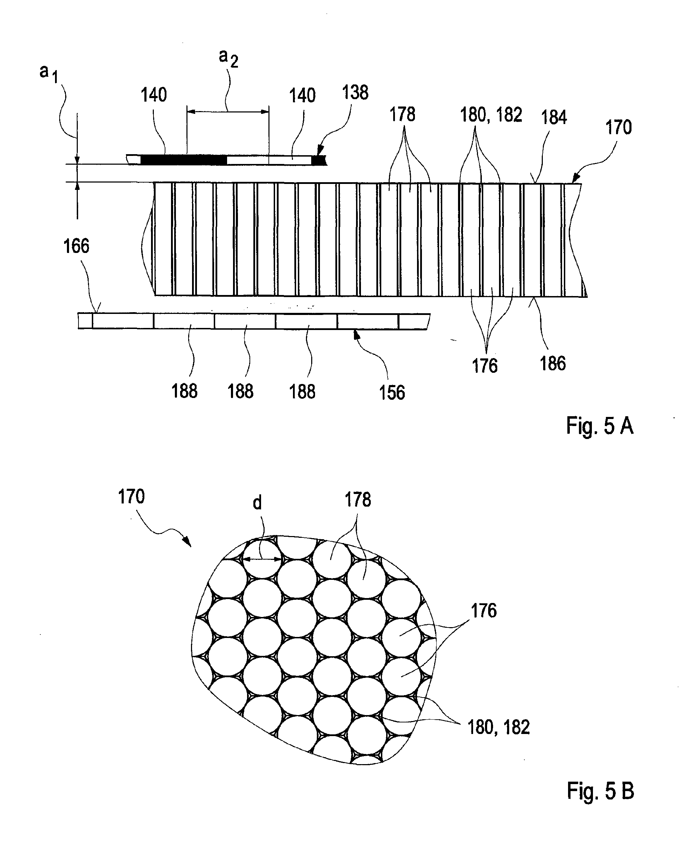

In den Figuren 5A und 5B ist in einer Schnittdarstellung von der Seite bzw. in einer ausschnittsweisen Draufsicht eine lichtoptische Faserplatte 170 beispielhaft dargestellt, anhand derer der Aufbau und das Funktionsprinzip eines derartigen Elements erläutert werden sollen. Die lichtoptische Faserplatte 170 umfasst eine Vielzahl optischer Fasern 176, welche vorzugsweise, wie in Figur 5B zu erkennen, in dichtester Packung nebeneinander angeordnet sind. Diese optischen Fasern 176 sind miteinander verklebt oder verschmolzen. Dabei können die Faserkerne 178 vorzugsweise noch vollständig voneinander separiert sein und lediglich in einer gemeinsamen Matrix 180 eingebettet sein. Diese Matrix 180 kann beispielsweise aus einem Cladding (Fasermantel) der ursprünglichen einzelnen optischen Fasern 176 in den Zwischenräumen zwischen den Faserkernen 178 zusammengesetzt sein.

Wie in Figur 5A zu erkennen, sind die optischen Fasern 176 vorzugsweise zumindest weitgehend parallel zueinander orientiert, wobei die Orientierung beispielsweise senkrecht zu zwei Oberflächen 184, 186 der lichtoptischen Faserplatte sein kann. Diese beiden Oberflächen umfassen eine codeseitige Oberfläche 184 und eine sensorseitige Oberfläche 186.

Im Gegensatz zu einem abbildenden System, welches auf einer Lichtbrechung an gekrümmten Oberflächen oder an Linsensystemen basiert, basiert die lichtoptische Faserplatte 170 auf einem Transport von Licht von der codeseitigen Oberfläche 184 zur sensorseitigen Oberfläche 186 oder umgekehrt durch interne Totalreflexion in den optischen Fasern 176. Dies bedeutet jedoch, dass ein unmittelbar vor der codeseitigen Oberfläche 184 angeordneter Gegenstand, beispielsweise das Bild des optischen Codes 138, virtuell durch die lichtoptische Faserplatte 170 einfach zur sensorseitigen Oberfläche 186 transportiert wird. Dies ist dadurch zu erklären, dass ein Punktstrahler, welcher unmittelbar vor dieser codeseitigen Oberfläche 184 angeordnet ist, durch die optischen Fasern 176 in einen virtuellen Punktstrahler umgewandelt wird, welcher sich auf der sensorseitigen Oberfläche 186 befindet. Da das Bild des optischen Codes 138 zumindest gedanklich aus derartigen Punktstrahlern zusammengesetzt werden kann, bedeutet dies, dass, wenn dieses Bild an der codeseitigen Oberfläche 184 oder unmittelbar vor dieser codeseitigen Oberfläche 184 angeordnet ist, dieses virtuell in ein Bild auf der sensorseitigen Oberfläche 186 umgewandelt wird.

In Figur 5A ist weiterhin dargestellt, wie eine bevorzugte Beabstandung zwischen optischem Code 138 und lichtoptischer Faserplatte 170 ausgestaltet ist. So weist jeder optische Code 138, wie anhand von Figur 1 erläutert, als kleinste optische Einheiten Module 140 auf. Der in Figur 5A mit a₁ bezeichnete Abstand zwischen der lichtoptischen Faserplatte 170 bzw. deren codeseitiger Oberfläche 184 und dem optischen Code 138 ist vorzugsweise kleiner als ein in Figur 5A mit a₂ bezeichneter Abstand zwischen den Mittelpunkten benachbarter Module 140.

Weiterhin ist in Figur 5A auch symbolisch eine bevorzugte Relation zwischen dem Bildsensor 156 und der Ausgestaltung der lichtoptischen Faserplatte 170 gezeigt. Der Bildsensor 156 setzt sich, wie oben beschrieben, aus einer Mehrzahl von Sensoren 188 zusammen, welche, wie in Figur 5A angedeutet, beispielsweise linear oder in einer zweidimensionalen Matrix angeordnet sein können. Die lichtoptische Faserplatte 170 ist vorzugsweise derart ausgestaltet, dass pro derartigem Sensor 188 und pro Dimension mindestens drei derartiger optischer Fasern 176 vorgesehen sind. Bei einer zweidimensionalen Anordnung ist also eine Mindestanzahl von neun optischen Fasern 176 bevorzugt. Eine weitere Vergrößerung der Anzahl der optischen Fasern 176 kann zu einer Verbesserung der Auslesequalität führen. Wie oben dargestellt, haben die optischen Fasern 176 vorzugsweise einen Durchmesser d (siehe Figur 5B) von weniger als 100 µm.

Das Ausführungsbeispiel des Codelesers 142 gemäß Figur 4 erfordert eine Anordnung der Beleuchtungseinrichtung 172 auf einer Rückseite des medizinischen Verbrauchsartikels 122. Dies ist beispielsweise bei einem in ein Blutzuckermessgerät 114 eingeschobenen Teststreifen 128 realisierbar, da dieser Teststreifen 128 zwischen die Beleuchtungseinrichtung 172 und die Sensorplatine 158 eingeschoben werden kann. In vielen Fällen ist diese Anordnung jedoch nachteilig, da zumeist gefordert wird, dass die Beleuchtungseinrichtung 172 und die Sensorplatine 158 eine Einheit bilden, beispielsweise eine einzelne elektronische Baugruppe. Die ist mit der Anordnung gemäß Figur 4 nur schwer realisierbar.

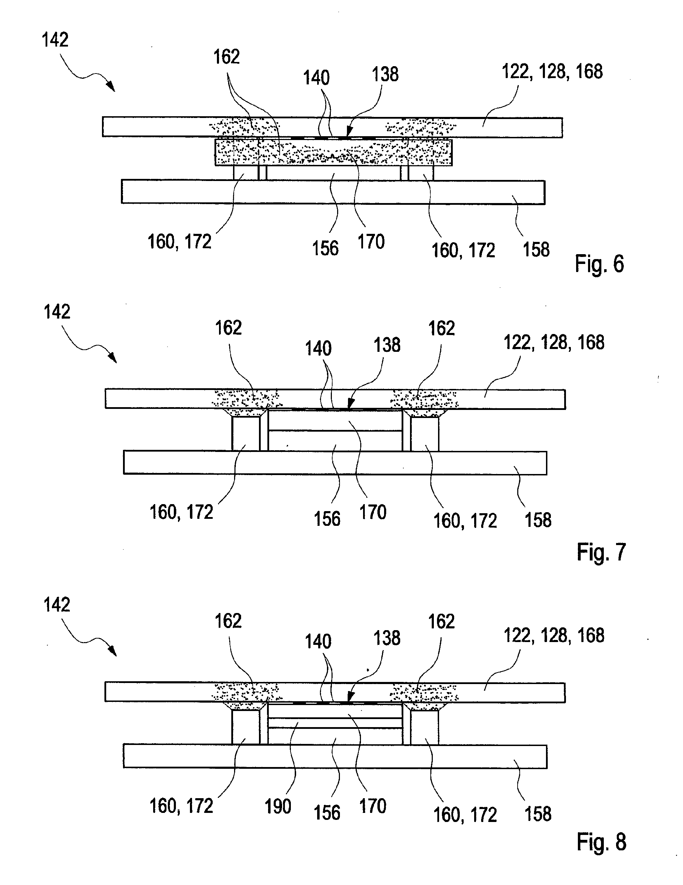

In den Figuren 6 bis 9 sind dementsprechend Ausführungsformen des Codelesers 142 dargestellt, welche eine derartige Ausgestaltung realisieren und in welchen die Beleuchtung des optischen Codes 138 durch die Beleuchtungseinrichtung 172 im Wesentlichen von derselben Seite des medizinischen Verbrauchsartikels 122 erfolgt wie die Detektion durch den Bildsensor 156.

In Figur 6 ist ein Ausführungsbeispiel gezeigt, bei welchem wiederum eine lichtoptische Faserplatte 170 unmittelbar oder mit lediglich geringem Abstand auf dem Bildsensor 156 aufliegt. Seitlich des Bildsensors 156 sind am Rand der lichtoptischen Faserplatte 170 und/oder in Aussparungen derselben Lichtquellen 160 vorgesehen, beispielsweise wiederum Leuchtdioden. Diese Lichtquellen 160 strahlen Beleuchtungslicht 162 seitlich in die lichtoptische Faserplatte 170 ein, also zumindest näherungsweise senkrecht zur Längserstreckung der optischen Fasern 176 in der lichtoptischen Faserplatte 170 und damit zur Beobachtungsrichtung des Bildsensors 156. Beispielsweise kann dieses Beleuchtungslicht 162 durch das Cladding 182 und/oder die Matrix 180 der lichtoptischen Faserplatte 170 eindringen, so dass insgesamt die lichtoptische Faserplatte 170 und damit auch der unmittelbar darüber angeordnete optische Code 138 beleuchtet werden. Alternativ oder zusätzlich kann das Beleuchtungslicht 162 auch in ein Trägermaterial 168 des medizinischen Verbrauchsartikels 122 eindringen, welches beispielsweise wiederum transparent oder zumindest mit teilweise lichtstreuenden Eigenschaften ausgestaltet sein kann. Dementsprechend kann eine Detektion des von dem optischen Code 138 reflektierten Lichts und/oder eine Durchleuchtung des optischen Codes 138, also eine Detektion des transmittierten Lichts, stattfinden. Alternativ oder zusätzlich kann, wie oben dargestellt, der optische Code 138 bzw. dessen Module 140 auch optische Eigenschaften wie Lumineszenzeigenschaften, Konversionseigenschaften oder Ähnliches aufweisen. Zu diesem Zweck kann beispielsweise eine lumineszierende Tinte aufgedruckt werden, es kann eine Laserkonversion eines Farbstoffs erfolgen oder Ähnliches. Als Trägermaterial 168 mit diffus das Beleuchtungslicht 162 leitenden Eigenschaften lässt sich beispielsweise ein Polyester einsetzen, welcher auch mit einer Dotierung versehen sein kann, beispielsweise einer Titandioxid-Dotierung, so dass das Trägermaterial 168 nach wie vor grundsätzlich einen weißen Eindruck vermittelt.

In dem in Figur 6 dargestellten Ausführungsbeispiel können, wie auch in den anderen erfindungsgemäßen Ausgestaltungen, auch ein oder mehrere zusätzliche Elemente vorgesehen sein. Insbesondere können ein oder mehrere Filter vorgesehen sein, beispielsweise Interferenzfilter und/oder Kantenfilter. So kann beispielsweise zwischen dem Bildsensor 156 und der lichtoptischen Faserplatte 170 ein Filter vorgesehen sein, um das Beleuchtungslicht 162 von dem eigentlichen Detektionslicht, welches vom optischen Code 138 ausgeht, zu trennen. Letzteres ist insbesondere in dem Fall von Vorteil, in welchem der optische Code 138 bzw. dessen Module 140 oder die für diese Module 140 verwendete Tinte bzw. Farbe, durch das Beleuchtungslicht 162 zu einer Emission von Detektionslicht angeregt werden, welches sich spektral von dem Beleuchtungslicht 162 unterscheidet. Auf diese Weise kann der Kontrast und das Signal-zu-Rausch-Verhältnis des von dem Bildsensor 156 aufgenommenen Signals erheblich verbessert werden.

In Figur 7 ist ein zu Figur 6 grundsätzlich ähnliches Ausführungsbeispiel des Codelesers 142 dargestellt. Wiederum sind die Lichtquellen 160 seitlich neben der lichtoptischen Faserplatte 170 angeordnet. Im Gegensatz zu dem Ausführungsbeispiel gemäß Figur 6 erfolgt jedoch in diesem Ausführungsbeispiel im Wesentlichen keine Einkopplung von Beleuchtungslicht 162 in die lichtoptische Faserplatte 170 von der Seite her, was beispielsweise durch eine geometrische Ausrichtung und/oder Direktionalität dieser Lichtquellen 160 realisiert werden kann und/oder durch entsprechende Abschirmelemente, beispielsweise lichtundurchlässige Farbgestaltung der Seitenkanten der lichtoptischen Faserplatte 170, beispielsweise durch eine Schwarzfärbung.

Das Beleuchtungslicht 162 wird dabei in dem Trägermaterial 168 des medizinischen Verbrauchsartikels 122 gestreut, wozu dieses Trägermaterial wiederum entsprechend ausgestaltet sein kann. Diese Streuung kann mit unveränderter Wellenlänge erfolgen, oder es können Streuzentren vorgesehen sein, welche wellenlängenkonvertierende Eigenschaften haben. Beispielsweise können wiederum Titandioxid-Partikel in dem Trägermaterial 168 vorgesehen sein, welches beispielsweise einen transparenten Kunststoff, beispielsweise wiederum ein Polyester, z.B. Melinex, umfassen kann. Aufgrund der Streueigenschaften dieser Streuzentren entsteht eine inhomogene Intensitätsverteilung in dem Matrixmaterial des Trägermaterials 168. Der optische Code 138 wird auf diese Weise von hinten beleuchtet, was einen erforderlichen Kontrast zwischen beispielsweise schwarzen und weißen Modulen 140 dieses optischen Codes 138 erzeugt. Alternativ oder zusätzlich zu dieser absorptiven Detektion kann jedoch auch wiederum eine Anregung des optischen Codes 138 durch das Beleuchtungslicht 162 erfolgen, beispielsweise wiederum eine Anregung zu einer Lumineszenz. Alternativ oder zusätzlich zur absorptiven Bildaufnahme kann auch dieses spektral veränderte Detektionslicht aufgenommen werden, beispielsweise durch eine spektrale Trennung in dem Bildsensor 156.

In Figur 8 ist ein weiteres Ausführungsbeispiel eines Codelesers 142 dargestellt, welches vom Aufbau her wiederum dem Ausführungsbeispiel gemäß Figur 7 ähnelt, so dass weitgehend auf die Beschreibung der Figur 7 verwiesen werden kann. Auch hier erfolgt wiederum vorzugsweise keine Einkopplung von Beleuchtungslicht 162 seitlich in die lichtoptische Faserplatte 170, sondern vorzugsweise ausschließlich eine Lichteinkopplung in ein Trägermaterial 168 des medizinischen Verbrauchsartikels 122.

Das in Figur 8 gezeigte Ausführungsbeispiel weist Vorteile gegenüber dem Ausführungsbeispiel gemäß Figur 7 hinsichtlich einer Unterdrückung von Untergrundlicht auf. Zu diesem Zweck wird Beleuchtungslicht 162 verwendet, welches durch den optischen Code 138 bzw. dessen Module 140 bzw. eine für diese Module verwendete Tinte bzw. Farbe spektral in seinen Eigenschaften verändert wird. Beispielsweise kann kurzwelliges Licht verwendet werden, welches ein Anregungslicht darstellt. Durch dieses Anregungslicht 162 kann beispielsweise in der Farbe bzw. Tinte des optischen Codes 138 eine Lumineszenz angeregt werden. Dieses Lumineszenzlicht, welches beispielsweise aufgrund einer Stokes-Verschiebung kürzerwellig ist als das Anregungslicht 162, kann durch geeignete spektralselektive Elemente vom Anregungslicht 162 getrennt werden. Exemplarisch ist zu diesem Zweck bei dem Aufbau gemäß Figur 8 zwischen der lichtoptischen Faserplatte und dem Bildsensor 156 ein Filter 190 vorgesehen, welches zwar für Detektionslicht zumindest weitgehend durchlässig ist, welches das Anregungslicht 162 jedoch zumindest weitgehend unterdrückt. Dieses Filter 190 kann beispielsweise direkt zwischen der lichtoptischen Faserplatte 170 und dem Bildsensor 156 vorgesehen sein. Alternativ oder zusätzlich sind jedoch auch andere Anordnungen möglich. Um zu vermeiden, dass die Bildqualität auf dem Bildsensor 156 durch die Dicke des Filters 190 verschlechtert wird, sind Dicken von lediglich wenigen 100 µm oder weniger für das Filter 190 bevorzugt. Beispielsweise kann eine Filterbeschichtung unmittelbar auf dem Bildsensor 156 bzw. dessen aktiver Sensoroberfläche 166 vorgesehen sein. Alternativ oder zusätzlich kann der Bildsensor 156 bzw. dessen Sensoren 188 auch selbst mit spektralselektiven Eigenschaften ausgestattet sein, beispielsweise mit einer spektralen Sensitivität lediglich im Bereich des zu detektierenden Detektionslichts.

Ein Vorteil der Verwendung fluoreszierender Tinte für den optischen Code 138 besteht weiterhin auch darin, dass der optische Code für einen Benutzer zumindest weitgehend unsichtbar ausgestaltet werden kann. Somit kann der optische Code 138 auch zur Fälschungssicherung verwendet werden, da dieser für einen Benutzer oder möglichen Nachahmer der medizinischen Verbrauchsartikel 122 nicht unmittelbar erkennbar ist.

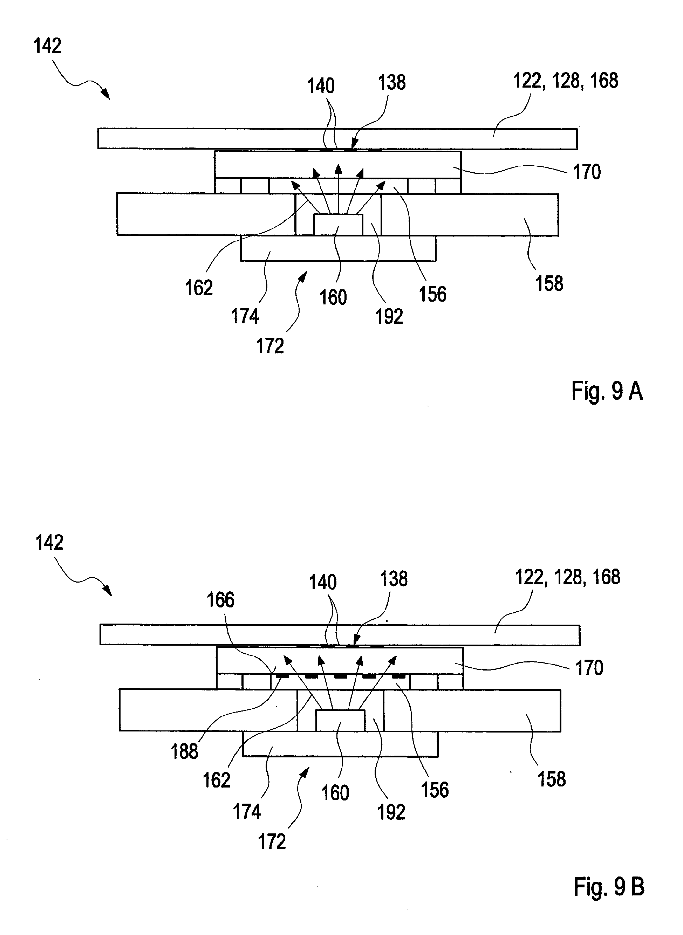

In Figur 9A ist ein weiteres Ausführungsbeispiel eines Codelesers 142 schematisch dargestellt, welches ein drittes, alternativ oder zusätzlich realisierbares Beleuchtungskonzept verfolgt. Während in Figur 4 eine rückseitige Durchleuchtung erfolgt und während in den Figuren 6 bis 8 eine seitliche Beleuchtung erfolgt, erfolgt bei dem Ausführungsbeispiel gemäß Figur 9A eine Beleuchtung mit Beleuchtungslicht 162 unmittelbar durch die lichtoptische Faserplatte 170 hindurch, wobei die Beleuchtung im Wesentlichen parallel zur Längserstreckung der optischen Fasern 176 erfolgt. Dies wird in dem dargestellten Ausführungsbeispiel dadurch realisiert, dass der Bildsensor 156 zumindest weitgehend transparent für das Beleuchtungslicht 162 ausgestaltet wird. Beispielsweise kann dies dadurch erfolgen, dass der Bildsensor 156 bzw. dessen Sensoren 188 ein Halbleitermaterial aufweisen, welches eine Bandlücke aufweist. Das Beleuchtungslicht 162 kann derart gewählt werden, dass dieses eine längere Wellenlänge aufweist als die dieser Bandlücke entsprechende Wellenlänge, so dass lediglich eine zu vernachlässigende Absorption des Beleuchtungslichts 162 in dem Bildsensor 156 erfolgt. In dem Trägermaterial 168 und/oder in dem Material des optischen Codes 138 können dann lichtkonvertierende Materialien vorgesehen sein, welche dieses langwellige Beleuchtungslicht 162 in entsprechend kürzerwelliges Detektionslicht umwandeln, welches dann wiederum von dem Bildsensor 156 wahrgenommen werden kann.

Alternativ oder zusätzlich zu der in Figur 9B beschriebenen Ausführungsform, bei welcher das Beleuchtungslicht 162 das Material des Bildsensors 156 unmittelbar durchdringt, kann der Bildsensor 156 auch speziell für einen Lichtdurchtritt ausgestaltete Bereiche umfassen. Dies ist in Figur 9B exemplarisch dargestellt, welche weitgehend der Figur 9A entspricht, so dass für die Beschreibung des Aufbaus weitgehend auf die obige Beschreibung der Figur 9A verwiesen werden kann.

So lassen sich die lichtdurchlässigen Bereiche in dem Bildsensor 156 auf verschiedene Weise ausgestalten. Beispielsweise können diese Bereiche durch entsprechende Öffnungen in dem Bildsensor 156 realisiert sein, durch welche das Beleuchtungslicht 162 dringen kann. Alternativ oder zusätzlich können, wie in dem Ausführungsbeispiel gemäß Figur 9B dargestellt, auch unbeschichtete Bereiche in dem Bildsensor 156 vorgesehen sein, durch welche der Lichtdurchtritt erfolgen kann. Letzteres lässt sich beispielsweise mit einem Array von Sensoren 188 realisieren, welche beabstandet zueinander auf einem transparenten Träger, beispielsweise einer Glasplatte, angeordnet sind. Durch die Zwischenräume zwischen den einzelnen Sensoren 188 kann dann Anregungslicht 162 zumindest weitgehend ungehindert hindurchtreten. Verschiedene andere Ausgestaltungen sind möglich. Das Beleuchtungslicht 162 kann dann wiederum auf verschiedene Weisen mit dem optischen Code 138 wechselwirken. Beispielsweise ist hier wiederum eine Reflexion, eine Absorption, eine Lumineszenzanregung oder Ähnliches zu nennen.

Bezüglich der Zwischenräume zwischen den einzelnen Sensoren 188 kann angemerkt werden, dass diese bei vielen Bildsensoren 156 ohnehin vorhanden sind. Das Verhältnis zwischen dem durch die Sensoren 188 gebildeten Anteil an einer aktiven Sensoroberfläche 166 und der gesamten aktiven Sensoroberfläche 166 wird allgemein als Füllfaktor bezeichnet. Konstruktiv bedingt liegt dieser Füllfaktor bei den meisten Bildsensoren 156 bei Werten von weniger als 100%. Im Rahmen der vorliegenden Erfindung kann dies nicht nur toleriert werden, sondern kann sogar bewusst ausgenutzt werden. So kann beispielsweise in den Zwischenräumen zwischen den Sensoren 188 bereits zumindest ein Teil einer Elektronik angeordnet werden. Beispielsweise kann dies ein Teil der für die Auswertung der Signale der Sensoren 188 erforderlichen Elektronik seien, beispielsweise eine Transistorelektronik, Verstärker, Dioden oder Kombinationen der genannten und/oder anderer Elemente. Diese Elektronik kann auch zumindest teilweise transparent ausgestaltet werden. In diesem Falle kann also zumindest ein Teil der Elektronik bereits auf und/oder unmittelbar unterhalb der aktiven Sensoroberfläche 166 angeordnet werden, beispielsweise in derselben Schichtebene, in welcher auch die Sensoren 188 angeordnet sind. Dadurch kann beispielsweise wiederum die gesamte Baugröße des Bildsensors 156 verringert werden, und es lassen sich kostengünstigere Bildsensoren 156, beispielsweise Halbleiter-Bildsensoren, einsetzen. Beispielsweise können Bildsensoren 156 verwendet werden, welche Füllfaktoren von ca. 25% aufweisen. Die Nachteile geringer Füllfaktoren, welche insbesondere in einer geringeren Lichtausbeute liegen, machen sich bei Bildsensoren 156 nicht in gleichem Maße bemerkbar, wie beispielsweise bei Kamerasystemen. Insofern können auch vergleichsweise geringe Füllfaktoren toleriert werden. Allgemein lassen sich als Bildsensoren 156 beispielsweise, wie oben beschrieben, CMOS-Strukturen einsetzen.

Bei den Anordnungen gemäß den Figuren 9A oder 9B kann beispielsweise die Sensorplatine 158 mit einer Öffnung 192 ausgestattet sein, in welche eine oder mehrere Lichtquellen 160 der Beleuchtungseinrichtung 172 hineinragen. Auch andere Ausgestaltungen, bei welchen die mindestens eine Lichtquelle 160 bzw. eine derartige Lichtquelle hinter dem Bildsensor 156 angeordnet sind bzw. ist, sind möglich.

Im Rahmen der vorliegenden Erfindung ist es bevorzugt, wenn das vollständige Bild des optischen Codes 138 auf die aktive Sensoroberfläche 166 abgebildet wird. Zu diesem Zweck kann beispielsweise die aktive Sensoroberfläche 166 mit einer geeigneten Größe ausgestattet werden. Alternativ oder zusätzlich ist es jedoch grundsätzlich auch möglich, die lichtoptische Faserplatte 170 mit verkleinernden oder vergrößernden Eigenschaften auszugestalten. Für eine Verkleinerung eines Bildes kann beispielsweise die Dichte der optischen Fasern 176 auf der sensorseitigen Oberfläche 186 der lichtoptischen Faserplatte 170 gegenüber der codeseitigen Oberfläche 184 verändert werden, beispielsweise kleiner ausgestaltet werden als auf der codeseitigen Oberfläche 184, wobei insgesamt die Anzahl der optischen Fasern 176 vorzugsweise gleich bleibt. Eine derartige vergrößernde oder in gleicher Weise verkleinernde Ausgestaltung der lichtoptischen Faserplatte 170 ist grundsätzlich auch für andere Anwendungen als die in dem Rahmen der vorliegenden Erfindung beschriebenen Anwendungen denkbar. Auf diese Weise kann jedoch im Rahmen der vorliegenden Erfindung die Fläche des optischen Codes 138 beispielsweise an die Größe der lichtoptischen Faserplatte 170 angepasst werden, so dass diese beispielsweise optimal ausgenutzt wird und die Baugröße und Kosten damit klein bzw. gering bleiben.

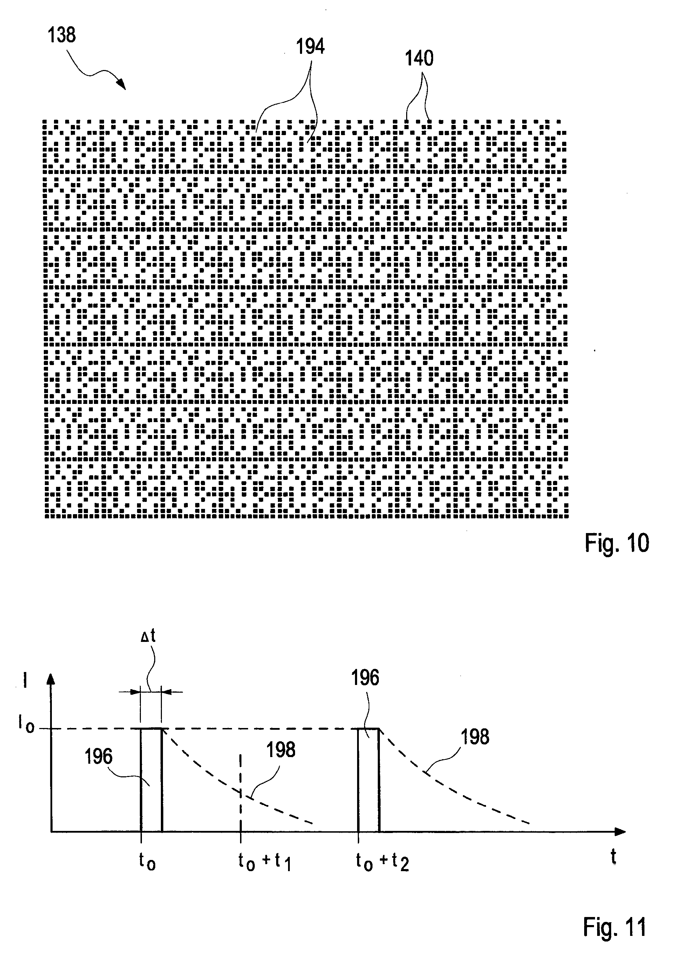

Alternativ ist es jedoch auch möglich, lediglich einen Teil des optischen Codes 138 durch den Bildsensor 156 zu erfassen. In diesem Fall ist es besonders bevorzugt, den optischen Code 138 mit redundanter Information auszugestalten. Ein Beispiel einer derartigen Ausgestaltung ist in Figur 10 dargestellt. Bei diesem Ausführungsbeispiel weist der optische Code 138 sich wiederholende, identische Codeeinheiten 194 auf. Diese Codeeinheiten 194 sind jeweils mit demselben Muster aus Modulen 140 ausgestaltet, welche erfasst werden können. Auf diese Weise ist es, auch wenn der Bildsensor 156 lediglich einen Ausschnitt des optischen Codes 138 erfasst, vorzugsweise ein Ausschnitt, welcher mindestens eine vollständige Codeeinheit 194 umfasst, möglich, die in diesem optischen Code 138 enthaltene Information auszulesen.

Allgemein kann die Auswertung der in dem optischen Code 138 enthaltenen Information, unabhängig von der Ausgestaltung des optischen Codes 138, bereits ganz oder teilweise in den Codeleser 142 erfolgen. Zu diesem Zweck kann beispielsweise der Bildsensor 156 mit einer eigenen Intelligenz ausgestattet werden, welche bereits eine teilweise oder vollständige Auswertung des optischen Codes 138 ermöglicht. Beispielsweise können in dem Bildsensor 156 bereits Filter, Bilderkennungsalgorithmen oder Ähnliches implementiert sein. Auch eine weitergehende Auswertung ist grundsätzlich möglich. Alternativ oder zusätzlich kann der Codeleser 142 auch zusätzliche elektronische Komponenten umfassen, welche weiterhin vollständig oder teilweise die Auswertung des optischen Codes 138 zur Gewinnung der mindestens einen darin enthaltenen Information realisieren. Diese zusätzlichen Bauelemente können beispielsweise ebenfalls auf der Sensorplatine 158 angeordnet sein oder können auch separat angeordnet sein. Wiederum alternativ oder zusätzlich kann eine weitere Auswertung auch von einer Steuereinheit des medizinischen Geräts 112 übernommen werden, beispielsweise einer zentralen Steuereinheit eines Blutzuckermessgeräts 114 oder einer Insulinpumpe 116. Verschiedene Ausgestaltungen sind möglich.

In den obigen Ausführungsbeispielen wurde die Beleuchtung des optischen Codes 138 mittels der Beleuchtungseinrichtung 172 stets als statisch betrachtet. Dies ist jedoch nicht notwendigerweise der Fall, sondern es kann, alternativ oder zusätzlich, auch eine zeitaufgelöste Beleuchtung und/oder Messung erfolgen. Ein Ausführungsbeispiel eines derartigen dynamischen oder zeitaufgelösten Messschemas ist in Figur 11 symbolisch gezeigt. Zahlreiche weitere Messschemata sind möglich.

In Figur 11 ist eine Intensität 1 des Beleuchtungslichts 162 gegen die Zeit t aufgetragen. Hieraus ist erkennbar, dass zu einem Zeitpunkt t₀ ein Beleuchtungspuls 196 mit einer Intensität I₀ emittiert wird. Dieser Beleuchtungspuls 196 kann, wie in Figur 11 gestrichelt angedeutet, beispielsweise einen Farbstoff des optischen Codes 138 zu einem Nachleuchten 198 anregen, welches länger andauert als die eigentliche Zeitdauer Δt des Beleuchtungspulses 196. Beispielsweise kann dieses Nachleuchten 198 eine Lumineszenz bzw. spezieller hier eine Phosphoreszenz umfassen.

Zu einem Zeitpunkt t₀ + t₁, wobei t₁ größer ist als Δt, kann dann eine Abfrage des Detektionslichts in Form des Nachleuchtens 198 durch den Bildsensor 156 erfolgen. Dies kann beispielsweise durch ein entsprechendes "Gate" in einer elektronischen Ansteuerung des Bildsensors erfolgen, welche durch den Beleuchtungspuls 196 getriggert wird. Auch diese Bildaufnahme kann, was in Figur 11 nicht dargestellt ist, wiederum über eine gewisse Zeitdauer erfolgen, so dass eine ausreichende Menge an Detektionslicht von dem Bildsensor 156 aufgenommen werden kann. Auf diese Weise kann durch das in Figur 11 gezeigte zeitliche Messschema durch ein Anregungs-Antwort-Messverfahren das Beleuchtungslicht 162 von dem Detektionslicht in Form des Nachleuchtens 198 getrennt werden, wodurch sich eine starke Verbesserung des Signal-zu-Rausch-Verhältnisses und eine Untergrundunterdrückung erzielen lässt.

Das Messverfahren kann auch wiederholt durchgeführt werden, was ebenfalls in Figur 11 angedeutet ist. So kann zu einem Zeitpunkt t₂, welcher größer ist als t₁, der Beleuchtungspuls 196 wiederholt werden, beispielsweise periodisch. Auf diese Weise kann das Messschema beispielsweise periodisch durchgeführt werden, so dass auch eine frequenzselektive Auswertung mittels eines Lock-in-Verfahrens realisierbar ist.

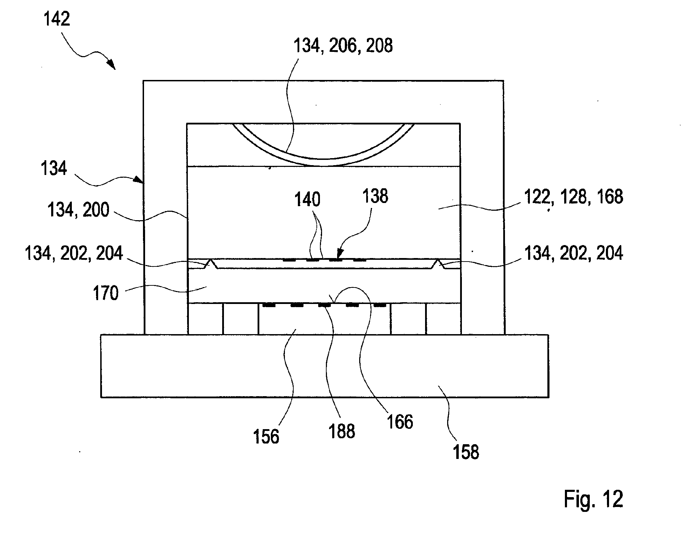

In Figur 12 ist ein zu den Figuren 4, 6 bis 8 und 9A und 9B alternatives Ausführungsbeispiel eines Codelesers 142 dargestellt, welches ein Beispiel einer möglichen Positionierungsvorrichtung 134 in einer schematischen Darstellung zeigt. Der Codeleser 142 kann dabei beispielsweise weitgehend wie die in den vorhergehenden Ausführungsbeispielen beschriebenen Codeleser 142 ausgestaltet sein, so dass für mögliche Ausführungsformen beispielsweise auf die obige Beschreibung verwiesen werden kann. Eine Beleuchtungseinrichtung 172 ist in Figur 12 nicht dargestellt. Die Beleuchtung kann ebenfalls beispielsweise wiederum wie in den zuvor beschriebenen Ausführungsbeispielen erfolgen.

Die Positionierungsvorrichtung 134 umfasst dabei in dem in Figur 12 dargestellten Ausführungsbeispiel optional einen Einschub 200, in welchen der medizinische Verbrauchsartikel 122 eingeschoben werden kann. Beispielsweise kann es sich bei diesen medizinischen Verbrauchsartikel 122 wiederum um ein Testelement 124, beispielsweise einen Teststreifen 128, handeln. Auch andere Arten medizinischer Verbrauchsartikel 122 sind jedoch wiederum möglich, wobei die Positionierungsvorrichtung 134 auf die geometrische Gestalt dieser Verbrauchsartikel 122 angepasst werden kann. So kann beispielsweise der Einschub 200 durch entsprechende andere Arten von Halterungen oder ähnlicher Vorrichtungen ersetzt werden.

Die Positionierungsvorrichtung 134 umfasst weiterhin in dem in Figur 12 dargestellten Ausführungsbeispiel optional einen Abstandshalter 202. Dieser Abstandshalter 202 umfasst beispielsweise eine Distanzschiene 204, welche eingerichtet ist, um einen vorgegebenen Mindestabstand zwischen dem medizinischen Verbrauchsartikel 122 und der lichtoptischen Faserplatte 170 zu gewährleisten. Auf diese Weise kann sichergestellt werden, dass die lichtoptische Faserplatte 170 durch den medizinischen Verbrauchsartikel 122, insbesondere beim Einschieben und/oder Ausschieben desselben, nicht beschädigt, abgenutzt oder verschmutzt wird. Der Mindestabstand kann beispielsweise zumindest weitgehend dem in Figur 5A mit a₁ bezeichneten Abstand entsprechen.

Weiterhin umfasst die Positionierungsvorrichtung 134 in dem in Figur 12 dargestellten Ausführungsbeispiel optional ein Andruckelement 206, welches hier beispielsweise als Federelement 208 angedeutet ist. Dieses Andruckelement 206 beaufschlagt den medizinischen Verbrauchsartikel 122 mit einer Kraft in Richtung der lichtoptischen Faserplatte 170. Auf diese Weise wird der medizinische Verbrauchsartikel 122 gegen die Distanzschiene 204 gepresst, so dass sichergestellt ist, dass der Abstand zwischen dem medizinischen Verbrauchsartikel 122 und der lichtoptischen Faserplatte 170 einen gewünschten Maximalabstand nicht überschreitet. Beispielsweise kann die Positionierungsvorrichtung 134 eingerichtet sein, um den medizinischen Verbrauchsartikel 122 in dem oben beschriebenen Abstand a₁ zu halten. Auch andere Ausgestaltungen der Positionierungsvorrichtung sind jedoch grundsätzlich möglich.

**Bezugszeichenliste**

| | | | |
|---|---|---|---|
| 110 | medizinisches System | 176 | optische Fasern |
| 112 | medizinisches Gerät | 178 | Faserkerne |
| 114 | Blutzuckermessgerät | 180 | Matrix |
| 116 | Insulinpumpe | 182 | Cladding |
| 118 | Eingabemittel | 184 | codeseitige Oberfläche |
| 120 | Ausgabemittel | 186 | sensorseitige Oberfläche |
| 122 | medizinische Verbrauchsartikel | 188 | Sensoren |
| 124 | Testelement | 190 | Filter |
| 126 | steifes Testelement | 192 | Öffnung |
| 128 | Teststreifen | 194 | Codeeinheiten |
| 130 | Applikationsstelle | 196 | Beleuchtungspuls |
| 132 | Eingabeöffnung | 198 | Nachleuchten |
| 134 | Positioniervorrichtung | 200 | Einschub |
| 136 | Elektrodenkontakte | 202 | Abstandshalter |
| 138 | optischer Code | 204 | Distanzschiene |
| 140 | Module | 206 | Andruckelement |
| 142 | Codeleser | 208 | Federelement |
| 144 | Insulinkartusche | | |
| 146 | Infusionsset | | |
| 148 | Schlauchkanüle | | |
| 150 | Adapter | | |
| 152 | Kanüle | | |
| 154 | Verpackung | | |
| 156 | Bildsensor | | |
| 158 | Sensorplatine | | |
| 160 | Lichtquelle | | |
| 162 | Beleuchtungslicht | | |
| 164 | Abbildungssystem | | |
| 166 | aktive Sensoroberfläche | | |
| 168 | Trägermaterial | | |
| 170 | lichtoptische Faserplatte | | |
| 172 | Beleuchtungseinrichtung | | |
| 174 | Beleuchtungsplatine | | |

## Patentansprüche

1. Medizinisches System (110), umfassend mindestens ein medizinisches Gerät (112) zur Durchführung mindestens einer medizinischen Funktion, insbesondere ein als Handgerät ausgestaltetes medizinisches Gerät (112), wobei das medizinische Gerät (112) eingerichtet ist, um zur Durchführung der medizinischen Funktion mit mindestens einem medizinischen Verbrauchsartikel (122) zusammenzuwirken, wobei das medizinische Gerät (112) mindestens einen Codeleser (142) zum Auslesen mindestens einer Information eines optischen Codes (138) auf dem medizinischen Verbrauchsartikel (122) aufweist, wobei der Codeleser (142) mindestens einen Bildsensor (156) mit einer Mehrzahl von Sensoren (188) umfasst, wobei der Codeleser (142) weiterhin mindestens eine lichtoptische Faserplatte (170) umfasst, wobei die lichtoptische Faserplatte (170) angeordnet ist, um ein Bild des optischen Codes (138) zu dem Bildsensor (156) zu leiten, weiterhin umfassend mindestens einen medizinischen Verbrauchsartikel (122), wobei der medizinische Verbrauchsartikel (122) mindestens einen von dem Codeleser (142) auslesbaren optischen Code (138) aufweist, wobei der optische Code (138) eine Mehrzahl von optisch lesbaren Modulen (140) als kleinste Informationseinheit aufweist, wobei der Codeleser (142) mindestens eine Beleuchtungseinrichtung (172) aufweist, **dadurch gekennzeichnet, dass** die lichtoptische Faserplatte (170) als ebene Platte ausgestaltet ist, wobei die lichtoptische Faserplatte (170) eingerichtet ist, um Licht von einer ersten Ebene der lichtoptischen Faserplatte (170) zu einer zweiten Ebene der lichtoptischen Faserplatte (170) zu transportieren, ohne hierbei ein Bild nennenswert zu verändern, wobei der optische Code (138) ein zweidimensionaler optischer Code (138) ist, wobei in einer Lesestellung, in welcher der Codeleser (142) die Information des zweidimensionalen optischen Codes (138) des medizinischen Verbrauchsartikels (122) auslesen kann, das medizinische Gerät (112) und der medizinische Verbrauchsartikel (122) derart relativ zueinander positioniert sind, dass ein Abstand zwischen der lichtoptischen Faserplatte (170) und dem optischen Code (138) kleiner ist als ein Abstand zwischen Mittelpunkten benachbarter Module (140) des Codes (138), die Beleuchtungseinrichtung (172) eingerichtet ist, um den optischen Code (138) des Verbrauchsartikels (122) einseitig von derselben Seite, auf welcher auch der Bildsensor (156) vorgesehen ist, zu beleuchten, wobei die Beleuchtungseinrichtung (172) auf mindestens eine der folgenden Arten ausgestaltet ist:
- die Beleuchtungseinrichtung (172) ist eingerichtet, um den optischen Code (138) durch die lichtoptische Faserplatte (170) hindurch zu beleuchten;
- die Beleuchtungseinrichtung (172) ist eingerichtet, um den optischen Code (138) von der Seite, vorzugsweise im Wesentlichen senkrecht zu einer Blickrichtung der lichtoptischen Faserplatte (170), zu beleuchten, insbesondere durch ein Trägermaterial (168) des medizinischen Verbrauchsartikels (122) hindurch;
- die Beleuchtungseinrichtung (172) umfasst mindestens eine Lichtquelle (160), wobei die Lichtquelle (160) eingerichtet ist, um den medizinischen Verbrauchsartikel (122) im Bereich des optischen Codes (138) durch den Bildsensor (156) hindurch zu beleuchten.

2. Medizinisches System (110) nach dem vorhergehenden Anspruch, wobei die lichtoptische Faserplatte (170) eine Mehrzahl von optischen Fasern (176) mit einem Durchmesser von weniger als 100 Mikrometern aufweist.

3. Medizinisches System (110) nach einem der vorhergehenden Ansprüche, wobei der Codeleser (142) mindestens eine Beleuchtungseinrichtung (172) aufweist, wobei die Beleuchtungseinrichtung (172) mindestens eine Lichtquelle (160) umfasst, welche eingerichtet ist, um den medizinischen Verbrauchsartikel (122) im Bereich des optischen Codes (138) zu durchleuchten.

4. Medizinisches System (110) nach einem der vorhergehenden Ansprüche, wobei der Codeleser (142) mindestens eine Beleuchtungseinrichtung (172) aufweist, wobei die Beleuchtungseinrichtung (172) eingerichtet ist, um den medizinischen Verbrauchsartikel (122) mit Licht verschiedener Wellenlängen zu beleuchten, insbesondere sequenziell.

5. Medizinisches System (110) nach einem der vorhergehenden Ansprüche, wobei der Codeleser (142) mindestens eine Beleuchtungseinrichtung (172) aufweist, wobei der Codeleser (142) eingerichtet ist, um das Bild des optischen Codes (138) zeitverzögert zu einer Beleuchtung durch die Beleuchtungseinrichtung (172) aufzunehmen.

6. Medizinisches System (110) nach einem der vorhergehenden Ansprüche, wobei das medizinische Gerät (112) mindestens eines der folgenden Geräte umfasst:
- ein Analysegerät (114) zum Nachweis mindestens eines Analyten in einer Probe, insbesondere ein Analysegerät (114) zum Nachweis mindestens eines Metaboliten in einer Körperflüssigkeit, wobei das Analysegerät (114) eingerichtet ist, um mit einem medizinischen Verbrauchsartikel (122) in Form mindestens eines Testelements (124) zum Nachweis des Analyten, insbesondere einem Teststreifen (128) oder einem Testband, zusammenzuwirken;
- eine Dosiervorrichtung zur Dosierung mindestens eines Medikaments, insbesondere eine Insulinpumpe (116), wobei die Dosiervorrichtung eingerichtet ist, um mit einem Verbrauchsartikel (122) in Form eines Katheters und/oder einer Kanüle (148, 152) zusammenzuwirken, wobei die Information vorzugsweise eine Information über ein Füllvolumen des Katheters und/oder der Kanüle (148, 152) umfasst.

7. Medizinisches System (110) nach einem der vorhergehenden Ansprüche, wobei der Bildsensor (156) mindestens zwei Bereiche mit unterschiedlicher spektraler Empfindlichkeit aufweist.

8. Medizinisches System (110) nach einem der vorhergehenden Ansprüche, weiterhin umfassend eine Positionierungsvorrichtung (134), welche eingerichtet ist, um das medizinische Gerät (112) und den Verbrauchsartikel (122) relativ zueinander zu positionieren.

9. Medizinisches System (110) nach einem der vorhergehenden Ansprüche, umfassend mindestens einen medizinischen Verbrauchsartikel (122), wobei der medizinische Verbrauchsartikel (122) mindestens einen von dem Codeleser (142) auslesbaren optischen Code (138) aufweist, wobei der optische Code (138) eine Mehrzahl von optisch lesbaren Modulen (140) als kleinste Informationseinheit aufweist, wobei die lichtoptische Faserplatte (170) derart dimensioniert ist, dass mindestens drei optische Fasern (176) pro Dimension des optischen Codes (138) pro Modul (140) vorgesehen sind.

10. Medizinisches System (110) nach einem der vorhergehenden Ansprüche, umfassend mindestens einen medizinischen Verbrauchsartikel (122), wobei der medizinische Verbrauchsartikel (122) mindestens einen von dem Codeleser (142) auslesbaren optischen Code (138) aufweist, wobei der medizinische Verbrauchsartikel (122) im Bereich des optischen Codes (138) ein Trägermaterial (168) umfasst, wobei das Trägermaterial (168) transparente oder lichtstreuende Eigenschaften aufweist, wobei das Trägermaterial (168) vorzugsweise einen transparenten Kunststoff, insbesondere ein Polyester, insbesondere Melinex, umfasst.

11. Medizinisches System (110) nach einem der vorhergehenden Ansprüche, umfassend mindestens einen medizinischen Verbrauchsartikel (122), wobei der medizinische Verbrauchsartikel (122) mindestens einen von dem Codeleser (142) auslesbaren optischen Code (138) aufweist, wobei der optische Code (138) eingerichtet ist, um mit Licht unterschiedlicher Wellenlängen unterschiedlich zu wechselwirken.

12. Medizinisches System (110) nach einem der vorhergehenden Ansprüche, umfassend mindestens einen medizinischen Verbrauchsartikel (122), wobei der medizinische Verbrauchsartikel (122) mindestens einen von dem Codeleser (142) auslesbaren optischen Code (138) aufweist, wobei der optische Code (138) zumindest teilweise lumineszierende Eigenschaften aufweist, wobei der optische Code (138) vorzugsweise eine Mehrzahl von optisch lesbaren Modulen (140) als kleinste Informationseinheit aufweist, wobei der optische Code (138), insbesondere die Module (140) des optischen Codes (138), vorzugsweise mindestens einen Lichtkonverter umfasst, insbesondere einen Phosphor, wobei der Lichtkonverter eingerichtet ist, um ein Anregungslicht in ein Licht mit einer von dem Anregungslicht verschiedenen Wellenlänge umzuwandeln, insbesondere in ein Licht mit längerer Wellenlänge als das Anregungslicht.

13. Medizinisches System (110) nach dem vorhergehenden Anspruch, wobei der transparente Kunststoff derart mit TiO₂ dotiert ist, dass ein im Wesentlichen weißer Gesamteindruck des Trägermaterials (168) entsteht, wobei das Trägermaterial (168) diffus lichtleitende Eigenschaften aufweist.

14. Medizinisches System (110) nach einem der vorhergehenden Ansprüche, umfassend mindestens einen medizinischen Verbrauchsartikel (122), wobei der medizinische Verbrauchsartikel (122) mindestens einen von dem Codeleser (142) auslesbaren optischen Code (138) aufweist, wobei der medizinische Verbrauchsartikel (122) im Bereich des optischen Codes (138) einen Träger mit einem Trägermaterial (168) umfasst, wobei der Träger weiterhin mindestens einen Lichtkonverter umfasst, wobei der Lichtkonverter eingerichtet ist, um ein Anregungslicht in ein Licht mit einer von dem Anregungslicht verschiedenen Wellenlänge umzuwandeln.

## Claims

1. Medical system (110), comprising at least one medical apparatus (112) for carrying out at least one medical function, in particular a medical apparatus (112) configured as a handheld apparatus, wherein the medical apparatus (112) is designed to interact with at least one medical consumable item (122) in order to carry out the medical function, wherein the medical apparatus (112) has at least one code reader (142) for reading out at least one information component of an optical code (138) on the medical consumable item (122), wherein the code reader (142) comprises at least one image sensor (156) having a plurality of sensors (188), wherein the code reader (142) furthermore comprises at least one light-optical fiber plate (170), wherein the light-optical fiber plate (170) is arranged in order to guide an image of the optical code (138) to the image sensor (156), furthermore comprising at least one medical consumable item (122), wherein the medical consumable item (122) has at least one optical code (138) which can be read out by the code reader (142), wherein the optical code (138) has a plurality of optically readable modules (140) as smallest information unit, wherein the code reader (142) has at least one illumination device (172), **characterized in that** the light-optical fiber plate (170) is configured as a planar plate, wherein the light-optical fiber plate (170) is designed to transport light from a first plane of the light-optical fiber plate (170) to a second plane of the light-optical fiber plate (170), without appreciably altering an image in the process, wherein the optical code (138) is a two-dimensional optical code (138), wherein, in a read position, in which the code reader (142) can read out the information of the two-dimensional optical code (138) of the medical consumable item (122), the medical apparatus (112) and the medical consumable item (122) are positioned relative to one another in such a way that a distance between the light-optical fiber plate (170) and the optical code (138) is less than a distance between midpoints of adjacent modules (140) of the code (138), the illumination device (172) is designed to illuminate the optical code (138) of the consumable item (122) on one side from the same side on which the image sensor (156) is also provided, wherein the illumination device (172) is configured in at least one of the following ways:
- the illumination device (172) is designed to illuminate the optical code (138) through the light-optical fiber plate (170);
- the illumination device (172) is designed to illuminate the optical code (138) from the side, preferably substantially perpendicularly to a viewing direction of the light-optical fiber plate (170), in particular through a carrier material (168) of the medical consumable item (122);
- the illumination device (172) comprises at least one light source (160), wherein the light source (160) is designed to illuminate the medical consumable item (122) in the region of the optical code (138) through the image sensor (156).

2. Medical system (110) according to the preceding claim, wherein the light-optical fiber plate (170) has a plurality of optical fibers (176) having a diameter of less than 100 micrometers.

3. Medical system (110) according to either of the preceding claims, wherein the code reader (142) has at least one illumination device (172), wherein the illumination device (172) comprises at least one light source (160) which is designed to transilluminate the medical consumable item (122) in the region of the optical code (138).

4. Medical system (110) according to any of the preceding claims, wherein the code reader (142) has at least one illumination device (172), wherein the illumination device (172) is designed to illuminate the medical consumable item (122) with light having different wavelengths, in particular sequentially.

5. Medical system (110) according to any of the preceding claims, wherein the code reader (142) has at least one illumination device (172), wherein the code reader (142) is designed to record the image of the optical code (138) in a time-delayed manner with respect to an illumination by the illumination device (172).

6. Medical system (110) according to any of the preceding claims, wherein the medical apparatus (112) comprises at least one of the following apparatuses:
- an analysis apparatus (114) for detecting at least one analyte in a sample, in particular an analysis apparatus (114) for detecting at least one metabolite in a body fluid, wherein the analysis apparatus (114) is designed to interact with a medical consumable item (122) in the form of at least one test element (124) for detecting the analyte, in particular a test strip (128) or a test tape;
- a metering device for metering at least one medicament, in particular an insulin pump (116), wherein the metering device is designed to interact with a consumable item (122) in the form of a catheter and/or a cannula (148, 152), wherein the information preferably comprises information about a filling volume of the catheter and/or of the cannula (148, 152).

7. Medical system (110) according to any of the preceding claims, wherein the image sensor (156) has at least two regions having a differing spectral sensitivity.

8. Medical system (110) according to any of the preceding claims, furthermore comprising a positioning device (134), which is designed to position the medical apparatus (112) and the consumable item (122) relative to one another.

9. Medical system (110) according to any of the preceding claims, comprising at least one medical consumable item (122), wherein the medical consumable item (122) has at least one optical code (138) which can be read out by the code reader (142), wherein the optical code (138) has a plurality of optically readable modules (140) as smallest information unit, wherein the light-optical fiber plate (170) is dimensioned in such a way that at least three optical fibers (176) are provided per dimension of the optical code (138) per module (140).

10. Medical system (110) according to any of the preceding claims, comprising at least one medical consumable item (122), wherein the medical consumable item (122) has at least one optical code (138) which can be read out by the code reader (142), wherein the medical consumable item (122) comprises a carrier material (168) in the region of the optical code (138), wherein the carrier material (168) has transparent or light-scattering properties, wherein the carrier material (168) preferably comprises a transparent plastic, in particular a polyester, in particular Melinex.

11. Medical system (110) according to any of the preceding claims, comprising at least one medical consumable item (122), wherein the medical consumable item (122) has at least one optical code (138) which can be read out by the code reader (142), wherein the optical code (138) is designed to interact differently with light having different wavelengths.

12. Medical system (110) according to any of the preceding claims, comprising at least one medical consumable item (122), wherein the medical consumable item (122) has at least one optical code (138) which can be read out by the code reader (142), wherein the optical code (138) has at least partly luminescent properties, wherein the optical code (138) preferably has a plurality of optically readable modules (140) as smallest information unit, wherein the optical code (138), in particular the modules (140) of the optical code (138), preferably comprises at least one light converter, in particular a phosphor, wherein the light converter is designed to convert an excitation light into a light having a different wavelength than the excitation light, in particular into a light having a longer wavelength than the excitation light.

13. Medical system (110) according to the preceding claim, wherein the transparent plastic is doped with TiO₂ in such a way that a substantially white overall impression of the carrier material (168) arises, wherein the carrier material (168) has diffusely light-conducting properties.

14. Medical system (110) according to any of the preceding claims, comprising at least one medical consumable item (122), wherein the medical consumable item (122) has at least one optical code (138) which can be read out by the code reader (142), wherein the medical consumable item (122) comprises a carrier having a carrier material (168) in the region of the optical code (138), wherein the carrier furthermore comprises at least one light converter, wherein the light converter is designed to convert an excitation light into a light having a different wavelength than the excitation light.

## Revendications

1. Système médical (110), comprenant au moins un appareil médical (112) pour accomplir au moins une fonction médicale, notamment un appareil médical (112) configuré en tant qu'appareil manuel, l'appareil médical (112) étant conçu pour, en vue d'accomplir la fonction médicale, coopérer avec au moins un article de consommation médical (122), l'appareil médical (112) présentant au moins un lecteur de code (142) pour lire au moins une information d'un code optique (138) sur l'article de consommation médical (122), le lecteur de code (142) comprenant au moins un capteur d'image (156) doté d'une pluralité de capteurs (188), le lecteur de code (142) comprenant en outre au moins une plaque de fibres optiques (170), la plaque de fibres optiques (170) étant disposée pour guider une image du code optique (138) jusqu'au capteur d'image (156), comprenant en outre au moins un article de consommation médical (122), l'article de consommation médical (122) présentant au moins un code optique (138) pouvant être lu par le lecteur de code (142), le code optique (138) présentant une pluralité de modules (140) lisibles optiquement en tant que plus petites unités d'information, le lecteur de code (142) présentant au moins un dispositif d'éclairage (172), **caractérisé en ce que** la plaque de fibres optiques (170) est réalisée sous la forme d'une plage plane, la plaque de fibres optiques (170) étant conçue pour transporter de la lumière d'un premier plan de la plaque de fibres optiques (170) vers un deuxième plan de la plaque de fibres optiques (170) sans provoquer ici une modification notable d'une image, le code optique (138) étant un code optique (138) bidimensionnel, l'appareil médical (112) et l'article de consommation médical (122) étant positionnés l'un par rapport à l'autre dans une position de lecture dans laquelle le lecteur de code (142) peut lire l'information du code optique (138) bidimensionnel de l'article de consommation médical (122) de telle sorte qu'un écart entre la plaque de fibres optiques (170) et le code optique (138) est inférieur à un écart entre les points centraux des modules (140) voisins du code (138), le dispositif d'éclairage (172) étant conçu pour éclairer le code optique (138) de l'article de consommation (122) d'un côté depuis le même côté sur lequel est également prévu le capteur d'image (156), le dispositif d'éclairage (172) étant configuré d'au moins l'une des manières suivantes :
- le dispositif d'éclairage (172) est conçu pour éclairer le code optique (138) à travers la plaque de fibres optiques (170) ;
- le dispositif d'éclairage (172) est conçu pour éclairer le code optique (138) par le côté, de préférence pour l'essentiel perpendiculairement à un sens d'observation de la plaque de fibres optiques (170), notamment à travers un matériau porteur (168) de l'article de consommation médical (122) ;
- le dispositif d'éclairage (172) comprend au moins une source de lumière (160), la source de lumière (160) étant conçue pour éclairer l'article de consommation médical (122) dans la zone du code optique (138) à travers le capteur d'image (156).

2. Système médical (110) selon la revendication précédente, avec lequel la plaque de fibres optiques (170) présente une pluralité de fibres optiques (176) ayant un diamètre inférieur à 100 micromètres.

3. Système médical (110) selon l'une des revendications précédentes, avec lequel le lecteur de code (142) présente au moins un dispositif d'éclairage (172), le dispositif d'éclairage (172) incluant au moins une source de lumière (160) qui est conçue pour éclairer par transparence l'article de consommation médical (122) dans la zone du code optique (138).

4. Système médical (110) selon l'une des revendications précédentes, avec lequel le lecteur de code (142) présente au moins un dispositif d'éclairage (172), le dispositif d'éclairage (172) étant conçu pour éclairer l'article de consommation médical (122) avec de la lumière à différentes longueurs d'onde, notamment séquentiellement.

5. Système médical (110) selon l'une des revendications précédentes, avec lequel le lecteur de code (142) présente au moins un dispositif d'éclairage (172), le lecteur de code (142) étant conçu pour capturer l'image du code optique (138) avec un retard dans le temps par rapport à un éclairage par le dispositif d'éclairage (172).

6. Système médical (110) selon l'une des revendications précédentes, avec lequel l'appareil médical (112) comprend au moins l'un des appareils suivants :
- un appareil d'analyse (114) pour déceler au moins un analyte dans un échantillon, notamment un appareil d'analyse (114) pour déceler au moins un métabolite dans un fluide corporel, l'appareil d'analyse (114) étant conçu pour coopérer avec un article de consommation médical (122) sous la forme d'au moins un élément de test (124) pour déceler l'analyte, notamment une plaquette de test (128) ou une bande de test ;
- un dispositif de dosage pour doser au moins un médicament, notamment une pompe à insuline (116), le dispositif de dosage étant conçu pour coopérer avec un article de consommation (122) sous la forme d'un cathéter et/ou d'une canule (148, 152), l'information comprenant de préférence une information sur un volume de remplissage du cathéter et/ou de la canule (148, 152).

7. Système médical (110) selon l'une des revendications précédentes, avec lequel le capteur d'image (156) présente au moins deux zones ayant des sensibilités spectrales différentes.

8. Système médical (110) selon l'une des revendications précédentes, comprenant en outre un dispositif de positionnement (134) qui est conçu pour positionner l'appareil médical (112) et l'article de consommation (122) l'un par rapport à l'autre.

9. Système médical (110) selon l'une des revendications précédentes, comprenant au moins un article de consommation médical (122), l'article de consommation médical (122) présentant au moins un code optique (138) lisible par le lecteur de code (142), le code optique (138) présentant une pluralité de modules (140) lisibles optiquement en tant que plus petites unités d'information, la plaque de fibres optiques (170) étant dimensionnée de telle sorte qu'au moins trois fibres optiques (176) sont prévues par module (140) par dimension du code optique (138).

10. Système médical (110) selon l'une des revendications précédentes, comprenant au moins un article de consommation médical (122), l'article de consommation médical (122) présentant au moins un code optique (138) lisible par le lecteur de code (142), l'article de consommation médical (122) comprenant dans la zone du code optique (138) un matériau porteur (168), le matériau porteur (168) présentant des propriétés transparentes ou de diffusion de la lumière, le matériau porteur (168) comprenant de préférence une matière plastique transparente, notamment un polyester, notamment du Melinex.

11. Système médical (110) selon l'une des revendications précédentes, comprenant au moins un article de consommation médical (122), l'article de consommation médical (122) présentant au moins un code optique (138) lisible par le lecteur de code (142), le code optique (138) étant conçu pour interagir différemment avec de la lumière à différentes longueurs d'onde.

12. Système médical (110) selon l'une des revendications précédentes, comprenant au moins un article de consommation médical (122), l'article de consommation médical (122) présentant au moins un code optique (138) lisible par le lecteur de code (142), le code optique (138) présentant au moins partiellement des propriétés luminescentes, le code optique (138) présentant de préférence une pluralité de modules (140) lisibles optiquement en tant que plus petites unités d'information, le code optique (138), notamment les modules (140) du code optique (138), incluant de préférence au moins un convertisseur de lumière, notamment un phosphore, le convertisseur de lumière étant conçu pour convertir une lumière d'excitation en une lumière ayant une longueur d'onde différente de celle de la lumière d'excitation, notamment en une lumière ayant une longueur d'onde plus longue que celle de la lumière d'excitation.

13. Système médical (110) selon la revendication précédente, avec lequel la matière plastique transparente est dopée avec du TiO₂ de telle sorte qu'il se produit une impression globale essentiellement blanche du matériau porteur (168), le matériau porteur (168) présentant des propriétés de conduction diffuse de la lumière.

14. Système médical (110) selon l'une des revendications précédentes, comprenant au moins un article de consommation médical (122), l'article de consommation médical (122) présentant au moins un code optique (138) lisible par le lecteur de code (142), l'article de consommation médical (122) comprenant dans la zone du code optique (138) un support avec un matériau porteur (168), le support comprenant en outre au moins un convertisseur de lumière, le convertisseur de lumière étant conçu pour convertir une lumière d'excitation en une lumière ayant une longueur d'onde différente de celle de la lumière d'excitation.
